(19) Europäisches Patentamt
European Patent Office
Office européen des brevets

(11) Veröffentlichungsnummer : **0 179 386**
**B1**

(12) # EUROPÄISCHE PATENTSCHRIFT

(45) Veröffentlichungstag der Patentschrift : **07.06.89**

(21) Anmeldenummer : **85113060.9**

(22) Anmeldetag : **15.10.85**

(51) Int. Cl.$^4$ : **C 07 D211/90**, C 07 D401/12, C 07 D401/04, C 07 D413/04, A 61 K 31/44

(54) **1,4-Dihydropyridin-hydroxyamine, Verfahren zu ihrer Herstellung sowie ihre Verwendung in Arzneimitteln.**

(30) Priorität : **26.10.84 US 664904**
**23.08.85 US 769181**

(43) Veröffentlichungstag der Anmeldung : **30.04.86 Patentblatt 86/18**

(45) Bekanntmachung des Hinweises auf die Patenterteilung : **07.06.89 Patentblatt 89/23**

(84) Benannte Vertragsstaaten : **AT BE CH DE FR GB IT LI NL SE**

(56) Entgegenhaltungen :
**EP-A- 0 002 231**
**EP-A- 0 088 276**
**EP-A- 0 151 006**
**WO-A-86 /026 40**
**DE-A- 2 629 892**

(73) Patentinhaber : **BAYER AG**

**D-5090 Leverkusen 1 Bayerwerk (DE)**

(72) Erfinder : **Triggle, David, Prof. Dr.**
**II Hampton Court**
**Buffalo N.Y., 14221 (US)**
Erfinder : **Schwenner, Eckhard, Dr.**
**Claudiusweg 3**
**D-5600 Wuppertal 1 (DE)**
Erfinder : **Kinast, Günther, Dr.**
**Am Eckbusch 15a**
**D-5600 Wuppertal 1 (DE)**
Erfinder : **Kazda, Stanislav, Dr.**
**Gellertweg 18**
**D-5600 Wuppertal 1 (DE)**
Erfinder : **Knorr, Andreas, Dr.**
**Pahlkestrasse 15**
**D-5600 Wuppertal 1 (DE)**
Erfinder : **Garthoff, Bernward, Dr.**
**Haendelstrasse 22**
**D-4010 Hilden (DE)**

**EP 0 179 386 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue 1,4-Dihydropyridin-hydroxyamine, ein Verfahren zu ihrer Herstellung sowie ihre Verwendung in Arzneimitteln, insbesondere in kreislaufbeeinflussenden Mitteln.

Es ist bereits bekannt geworden, daß man 1,4-Dihydro-2,6-dimethyl-4-phenyl-pyridin-3,5-dicarbonsäurediethylester erhält, wenn man 2-Benzylidenacetessigsäureethylester mit β-Aminocrotonsäureethylester oder Acetessigsäureethylester und Ammoniak umsetzt (E. Knoevenagel, Ber. dtsch. chem. Ges. 31, 743 (1898)).

Weiterhin ist bekannt, daß bestimmte 1,4-Dihydropyridine interessante pharmakologische Eigenschaften aufweisen (F. Bossert, W. Vater, Naturwissenschaften 58, 578 (1971)).

Ebenso ist es aus den deutschen Offenlegungsschriften 28 47 236 und 22 18 644 bekannt, daß Dihydropyridinester als Coronarmittel und als blutdrucksenkende Mittel verwendet werden können.

In der nachpublizierten EP-A 151 006 werden ähnliche Dihydropyridinhydroxyamine beschrieben und beansprucht, die jedoch von den erfindungsgemäß beanspruchten Verbindungen eindeutig abgegrenzt sind. Insbesondere werden in dieser Entgegenhaltung keine Verbindungen beschrieben, in denen der Substituent A für eine spezifisch verzweigte Alkylengruppe steht oder für eine beliebige Alkylengruppe steht, wenn die restlichen Substituenten eine andere Bedeutung haben. Diese Entgegenhaltung ist somit im Hinblick auf Artikel 54(3) EPÜ nicht relevant.

Die vorliegende Erfindung betrifft 1,4-Dihydropyridine mit substituierten Carbonsäurefunktionen der allgemeinen Formel I

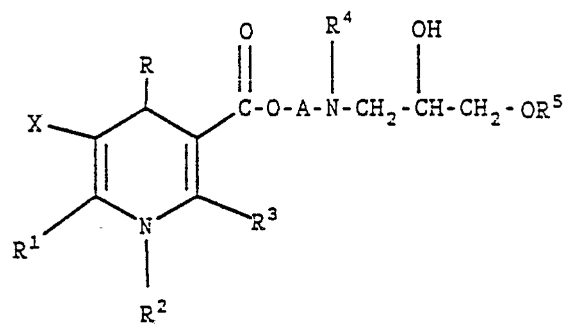

(I)

in welcher

R für Phenyl, Pyridyl oder den Benzoxadiazolylrest steht, wobei der Phenylrest 1 oder 2 gleiche oder verschiedene Substituenten aus der Gruppe Nitro, Trifluormethyl, Fluor, Chlor oder Cyano enthält,

$R^1$ und $R^3$ gleich oder verschieden sind und jeweils für Alkyl mit 1 bis 4 Kohlenstoffatomen stehen oder einer der Substituenten $R^1$ oder $R^3$ für Hydroxymethyl, Amino oder Cyano steht,

$R^2$ für Wasserstoff oder Alkyl mit 1 bis 2 Kohlenstoffatomen steht,

A entweder für eine durch Methyl, Ethyl oder Propyl substituierte Alkylengruppe mit bis zu 12 Kohlenstoffatomen steht, die gegebenenfalls durch eine Phenylgruppe substituiert oder unterbrochen ist und/oder die gegebenenfalls durch ein Sauerstoff- oder Schwefelatom oder eine Gruppierung der Formel $—NR^6$ unterbrochen ist,

oder

A für eine geradkettige oder verzweigte Alkylengruppe mit bis zu 12 Kohlenstoffatomen steht, die gegebenenfalls durch 1 Sauerstoff- oder Schwefelatom oder eine Gruppierung der Formel $—NR^6$ unterbrochen ist, wenn

a) $R^4$ für Phenyl, Benzyl, $CO—R^6$, $CO_2—R^{6'}$, $CO—NR^6R^7$, $SO_2R^6$ oder $—CH_2—CHOH—CH_2—OR^8$ steht, oder

b) R für Pyridyl oder Benzoxadiazolyl steht, oder

c) $R^5$ für Indolyl steht, oder

d) die Alkylenkette durch ein Schwefelatom oder die Gruppe $—NR^6$ unterbrochen ist oder

e) X für $COR^9$ steht,

wobei

$R^6$ und $R^7$ gleich oder verschieden sind und jeweils für Wasserstoff, Alkyl mit bis zu 4 C-Atomen, Benzyl oder Phenyl stehen und wobei

$R^8$ die für $R^5$ angegebene Bedeutung hat und mit diesen gleich oder verschieden sein kann und wobei

$R^9$ für Alkyl mit 1 bis 7 C-Atomen steht, welches gegebenenfalls durch Halogen, Cyano, Alkoxy oder Dialkylamino mit jeweils 1-4 C-Atomen substituiert ist, oder Phenyl, Naphthyl, Phenylalkyl oder Anilino oder eine Amino-, Monoalkylamino- oder Dialkylaminogruppe mit je 1 bis 4 C-Atomen bedeutet, wobei gegebenenfalls die Alkylgruppen mit dem Stickstoffatom einen 5- bis 7-gliedrigen Ring bilden, der als weiteres Heteroatom ein Sauerstoffatom enthalten kann und wobei

$R^{6'}$ die Bedeutung von $R^6$ hat jedoch nicht Wasserstoff bedeutet,

$R^4$ für Wasserstoff, für geradkettiges oder verzweigtes Alkyl mit 1 bis 3 Kohlenstoffatomen, für Phenyl, Benzyl oder für einen Rest der Formel $CO—R^6$, $CO_2—R^{6'}$, $CO—NR^6R^7$, $SO_2R^6$ oder $—CH_2—CHOH—CH_2—OR^8$ steht, wobei

$R^6$, $R^{6'}$, $R^7$, $R^8$ und $R^9$ die oben angegebene Bedeutung haben,

$R^5$ für Phenyl, Naphthyl oder Indolyl steht, wobei der Phenylrest gegebenenfalls durch 1 oder 2 gleiche oder verschiedene Substituenten aus der Gruppe Fluor, Chlor, Nitro, Cyano, Alkyl mit 1 bis 2 C-Atomen, $NR^6$, $R^7$, $NR^6COR^7$, die Gruppe $CH_2—CO—NH_2$ oder Acetyl substituiert ist und

X für die Gruppe $COOR^{10}$ steht, wobei $R^{10}$ für einen geradkettigen, verzweigten oder cyclischen Alkylrest mit bis zu 12 C-Atomen steht, der gegebenenfalls durch ein Sauerstoffatom in der Kette unterbrochen ist und/oder der gegebenenfalls substituiert ist durch Fluor, Chlor, Hydroxy, Pyridyl, Phenyl oder Amino oder für die Gruppe $COR^9$ steht, worin $R^9$ die oben angegebene Bedeutung hat, sowie deren pharmazeutische unbedenklichen Säureadditionssalze.

Die Herstellung der erfindungsgemäßen Verbindungen der allgemeinen Formel (I) erfolgt dadurch, daß man

Dihydropyridine der allgemeinen Formel II

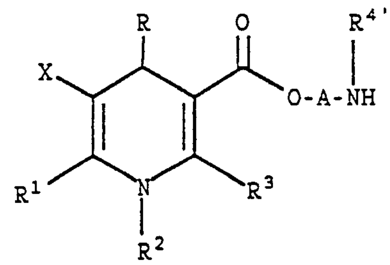

(II)

in welcher

R, $R^1$, $R^2$, $R^3$, X und A die oben angegebene Bedeutung haben, und

$R^{4'}$ für Wasserstoff, Alkyl, Aryl oder Aralkyl steht,

mit Epoxiden der allgemeinen Formel III

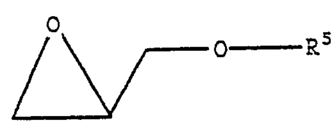

(III)

in welcher $R^5$ die oben angegebene Bedeutung hat,
gegebenenfalls in Gegenwart inerter organischer Lösungsmittel umsetzt.

Die Herstellung von Verbindungen der allgemeinen Formel (I), in welcher $R^4$ für den Rest

$$CH_2-\underset{OH}{CH}-CH_2-OR^8$$

steht, wobei $R^8$ von $R^5$ verschieden ist, erfolgt vorzugsweise durch stufenweise Umsetzung von primären Dihydropyridinaminen mit zunächst einem Epoxid mit dem Substituenten $R^5$ (IIIa) und anschließend einem weiteren Epoxid mit dem Substituenten $R^8$ (IIIb).

Die Herstellung von Verbindungen der allgemeinen Formel (I), in welcher $R^4$ für die Reste $CO—R^6$, $CO_2R^{6'}$, $CO—NR^6R^7$ oder $SO_2R^{6'}$ steht, erfolgt durch Umsetzung der Verbindungen (I) in welchen $R^4$ = Wasserstoff ist mit Acylierungs- oder Sulfonylierungsmitteln wie $R^6CO_2H$, $R^6—COCl$, $(R^{6'}CO)_2O_6$, $R^6O_2CCl$, $R^6R^7N—COCl$, $R^6—NCO$, $R^6SO_2Cl$, in welchen $R^6$, $R^{6'}$, $R^7$ die oben angegebene Bedeutung haben.

Das erfindungsgemäße Verfahren kann durch die in den folgenden Formelschemata angeführten Beispiele verdeutlicht werden.

(Siehe Formeln Seite 4 ff.)

a)

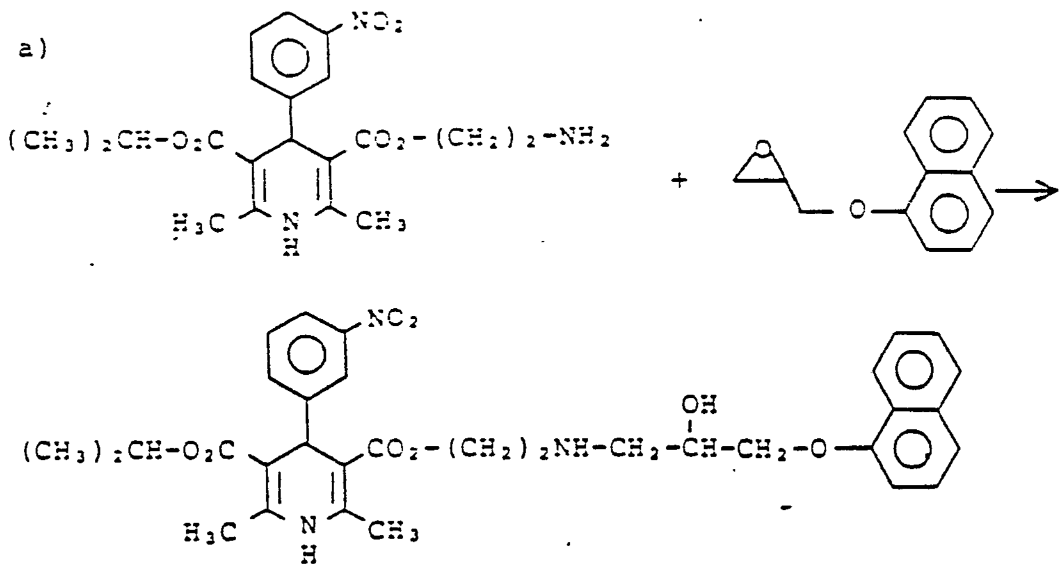

b)

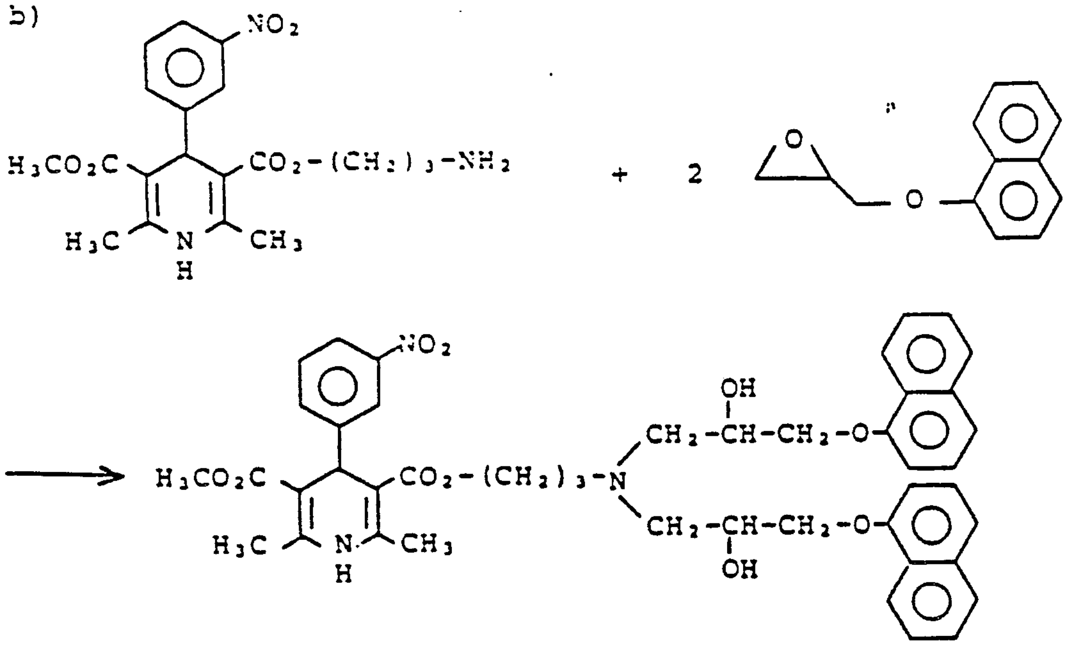

c)

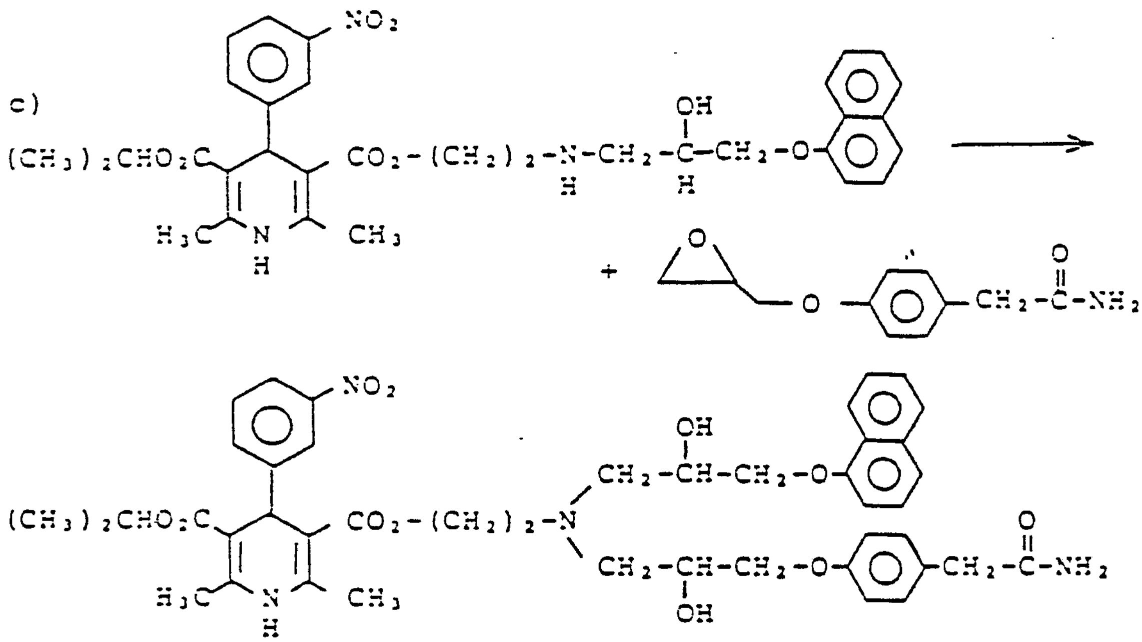

(Fortsetzung)

d)

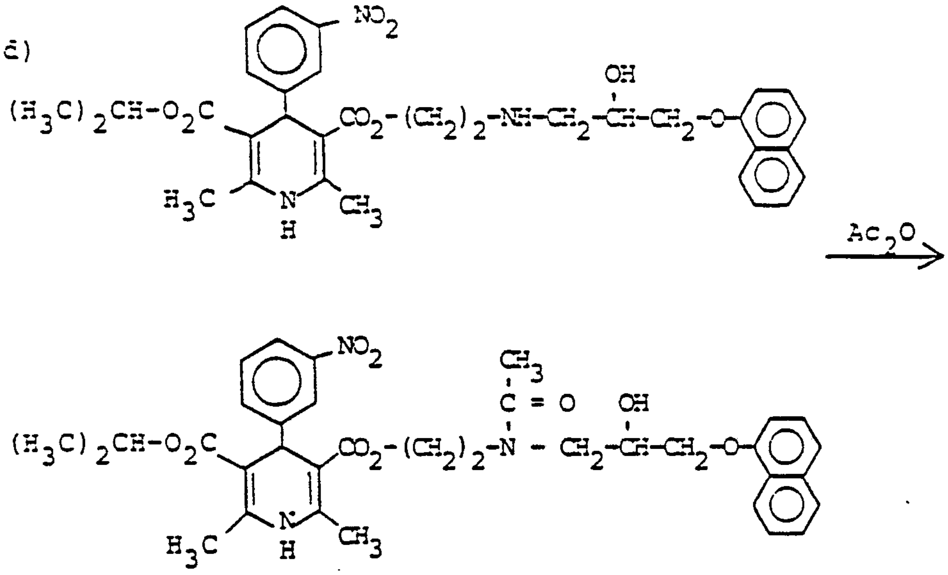


Als Säureadditionssalze seien vorzugsweise Salze mit organischen Säuren wie z. B. Maleinsäure, Fumarsäure oder Weinsäure, oder mit anorganischen Säuren wie z. B. Halogenwasserstoffsäuren oder Schwefelsäure genannt.

Die als Ausgangsstoffe verwendeten Dihydropyridinamine der allgemeinen Formel (II) sind literaturbekannt oder können nach literaturbekannten Verfahren, die sich im wesentlichen an die Hantzsche Pyridinsynthese anlehnen, hergestellt werden (vgl. EP-A 88 276).

Als Beispiele seien genannt:

1,4-Dihydro-2,6-dimethyl-4-(3-nitrophenyl)-pyridin-3,5-dicarbonsäure-isopropyl-(2-aminoethyl)-ester.

1,4-Dihydro-2,6-dimethyl-4-(2-nitrophenyl)-pyridin-3,5-dicarbonsäure-methyl-(3-aminopropyl)-ester.

1,4-Dihydro-2,6-dimethyl-4-(3-nitrophenyl)-pyridin-3,5-dicarbonsäure-(2-cyanoethyl)-(11-aminoundecyl)-ester.

1,4-Dihydro-2,6-dimethyl-4-(3-nitrophenyl)-pyridin-3,5-dicarbonsäure-neopentyl-(6-aminohexyl)-ester.

1,4-Dihydro-2,6-dimethyl-4-(3-nitrophenyl)-pyridin-3,5-dicarbonsäure-isobutyl-(5-aminopentyl)-ester.

1,4-Dihydro-2,6-dimethyl-4-(3-nitrophenyl)-pyridin-3,5-dicarbonsäure-methoxyethyl-(5-amino-2,2-dimethylpentyl)-ester.

1,4-Dihydro-2,6-dimethyl-4-(3-nitrophenyl)-pyridin-3,5-dicarbonsäure-ethyl-(2-N-methylaminoethyl)-ester.

1,4-Dihydro-2,6-dimethyl-4-(2-chlorphenyl)-pyridin-3,5-dicarbonsäure-isopropyl-(3-aminopropyl)-ester.

1-Ethyl-1,4-dihydro-2,6-dimethyl-4-(2,3-dichlorphenyl)-pyridin-3,5-dicarbonsäure-methyl-(2-N-benzylaminoethyl)-ester.

1,4-Dihydro-2,6-dimethyl-4-(2,3-dichlorphenyl)-pyridin-3,5-dicarbonsäure-methyl-(4-aminobutyl)-ester.

1,4-Dihydro-2,6-dimethyl-4-(2-trifluormethylphenyl)-pyridin-3,5-dicarbonsäure-cyclopentyl-(4-aminobutyl)-ester.

1,4-Dihydro-2,6-dimethyl-4-(3-pyridyl)-pyridin-3,5-dicarbonsäure-2,2,2-trifluorethyl-(5-aminopentyl)-ester.

1,4-Dihydro-2,6-dimethyl-4-(4-benzoxadiazolyl)-pyridin-3,5-dicarbonsäure-methyl-(2-aminoethyl)-ester.

1,4-Dihydro-2,6-dimethyl-4-(3-cyanoethyl)-pyridin-3,5-dicarbonsäure-(2-hydroxyethyl)-(3-aminopropyl)-ester.

1,4-Dihydro-2,6-dimethyl-4-(3-nitrophenyl)-pyridin-3,5-dicarbonsäure-neopentyl-(4-aminobutyl)-ester.

1,4-Dihydro-2,6-dimethyl-4-(2-trifluormethylphenyl)-pyridin-3,5-dicarbonsäure-ethoxyethyl-(6-aminohexyl)-ester.

1,4-Dihydro-2,6-dimethyl-4-(3-nitrophenyl)-pyridin-3,5-dicarbonsäure-allyl-(2-aminoisopropyl)-ester.

Die als Ausgangsstoffe verwendeten Epoxide der allgemeinen Formel (III) sind literaturbekannt oder können nach literaturbekannten Methoden hergestellt werden (vgl. A. F. Growther and L. H. Gnuth, J. Med. Chem. 11, 1009 (1968)).

Als Beispiele seien genannt:
1-Phenoxy-2,3-epoxy-propan.
1-(4-Methoxyphenoxy)-2,3-epoxy-propan.
1-(3-Methoxyphenoxy)-2,3-epoxy-propan.
1-(3-Methoxyphenoxy)-2,3-epoxy-propan.
1-(2-Allylphenoxy)-2,3-epoxy-propan.
1-(2-Allyloxyphenoxy)-2,3-epoxy-propan.
1-(4-Carbazolyloxy)-2,3-epoxy-propan.
1-(4-Methoxyethylphenoxy)-2,3-epoxy-propan.
1-(4-Indolyloxy)-2,3-epoxy-propan.
1-(1-Naphthyloxy)-2,3-epoxy-propan.
1-(4-Allyl-3-methoxyphenoxy)-2,3-epoxy-propan.
1-(2-Cyanophenoxy)-2,3-epoxy-propan.
1-(5-Allyl-2-ethoxyphenoxy)-2,3-epoxy-propan.
1-(4-Aminocarbonylmethyl-phenoxy)-2,3-epoxy-propan.
1-(4-Methylsulfonyl-N-methylamido-phenoxy)-2,3-epoxypropan.
1-(4-Methyl-carbaminoylethyl-phenoxy)-2,3-epoxy-propan.
1-(2-Acetyl-4-propionamido-phenoxy)-2,3-epoxy-propan.
1-(4-Acetamidophenoxy)-2,3-epoxy-propan.

Für das erfindungsgemäße Verfahren kommen alle inerten organischen Lösungsmittel in Frage. Hierzu gehören vorzugsweise niedere Alkohole wie Ethanol, Methanol, Propanol oder Isopropanol, Ether wie Dioxan, Diethylether, Tetrahydrofuran oder Glykolmonomethylether sowie chlorierte Kohlenwasserstoffe wie Dichlormethan, Chloroform und 1,2-Dichlorethan, sowie Gemische dieser Lösungsmittel.

Die Reaktionstemperaturen können in einem größeren Bereich variiert werden. Im allgemeinen arbeitet man zwischen 10 und 150 °C, vorzugsweise zwischen 20 und 130 °C, insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Für die Synthese der Verbindungen der Formel (I) mit $R^4$ = Wasserstoff, werden die entsprechenden Amine der Formel (II) in molaren bis doppelt molaren Mengen eingesetzt.

Für die weitere Reaktion zu Verbindungen der Formel (I), in welcher $R^4$ für den Rest

$$-CH_2-\underset{OH}{CH}-CH-OR^8$$

steht, werden die entsprechenden Epoxide der Formel (III) in molaren bis dreifach molaren Mengen eingesetzt.

Für die Acylierung bzw. Sulfonylierung werden die Reaktanden in molaren Mengen eingesetzt.

Das vorstehende Herstellungsverfahren ist lediglich zur Verdeutlichung angegeben, und die Herstellung der Verbindungen der Formel (I) ist nicht auf dieses Verfahren beschränkt, sondern jede Modifikation dieses Verfahrens ist in gleicher Weise für die Herstellung der erfindungsgemäßen Verbindungen anwendbar.

Je nach Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z. B. E. L. Eliel, Stereochemistry of Carbon Compounds, McGraw Hill, 1962).

Außer den unten angeführten Herstellungsbeispielen seien besonders noch folgende erfindungsgemäßen Wirkstoffe genannt:

1,4-Dihydro-2,6-dimethyl-4-(2-nitrophenyl)-pyridin-3,5-dicarbonsäure-isopropyl-{2-[hydroxy-3-(1-naphthyloxy)-1-propyl]-aminoethyl}-ester.

1,4-Dihydro-2,6-dimethyl-4-(2-trifluormethylphenyl)-pyridin-3,5-dicarbonsäure-cyclopentyl-{4-[2-hydroxy-3-(4-indolyloxy)-1-propyl]-aminobutyl}-ester.

1,4-Dihydro-2,6-dimethyl-4-(2,3-dichlorphenyl)-pyridin-3,5-dicarbonsäure-methyl-{6-[2-hydroxy-3-(4-aminocarbonylmethyl-phenoxy)-2-propyl]-aminohexyl}-ester.

1,4-Dihydro-2,6-dimethyl-4-(3-pyridyl)-pyridin-3,5-dicarbonsäure-isopropyl-{5-[2-cyanophenoxy)-1-propyl]-amino-2,2-dimethylpentyl}-ester.

1,4-Dihydro-2,6-dimethyl-4-(2-chlorphenyl)-pyridin-3,5-dicarbonsäure-(2,2-dimethyl)-ethyl-{3-[2-hydroxy-3-(4-methoxyethyl-phenoxy)-1-propyl]-aminopropyl}-ester.

1,4-Dihydro-2,6-dimethyl-4-(4-benzoxadiazolyl)-pyridin-3,5-dicarbonsäure-neopentyl-{2-[2-hydroxy-3-(2-allylphenoxy)-1-propyl]-N-methyl-aminoethyl}-ester.

1,4-Dihydro-2,6-dimethyl-4-(2-nitrophenyl)-pyridin-3,5-dicarbonsäure-isopropyl-{2-N,N-bis[2-hydroxy-3-(1-naphthyloxy)-1-propyl]-aminoethyl}-ester.

1,4-Dihydro-2,6-dimethyl-4-(2-trifluormethylphenyl)-pyridin-3,5-dicarbonsäure-cyclopentyl-{4-N,N-bis-[2-hydroxy-3-(4-indolyloxy)-1-propyl]-aminobutyl}-ester.

1,4-Dihydro-2,6-dimethyl-4-(2,3-dichlorphenyl)-pyridin-3,5-dicarbonsäure-methyl-{6-N,N-bis[2-hydroxy-3-(4-aminocarbonyl-methylphenoxy)-1-propyl]-aminohexyl}-ester.

1,4-Dihydro-2,6-dimethyl-4-(3-pyridyl)-pyridin-3,5-dicarbonsäure-isopropyl-{5-N,N-bis[2-hydroxy-3-(2-cyanophenoxy)-1-propyl]-amino-2,2-dimethylpentyl}-ester.

1,4-Dihydro-2,6-dimethyl-4-(2-chlorphenyl)-pyridin-3,5-dicarbonsäure-(2,2-dimethyl)-ethyl-{3-N,N-bis-[2-hydroxy-3-(4-methoxyethylphenoxy)-1-propyl]-aminopropyl}-ester.

1,4-Dihydro-2,6-dimethyl-4-(3-nitrophenyl)-pyridin-3,5-dicarbonsäure-(2-cyano)-ethyl-{2-N,N-bis[2-hydroxy-3-(4-indolyloxy)-1-propyl]-aminoethyl}-ester.

1,4-Dihydro-2,6-dimethyl-4-(2-nitrophenyl)-pyridin-3,5-dicarbonsäure-isobutyl-{5-N,N-bis[2-hydroxy-3-(1-naphthyloxy)-1-propyl]-aminopentyl}-ester.

1,4-Dihydro-2,6-dimethyl-4-(2-trifluormethyl-phenyl)-pyridin-3,5-dicarbonsäure-(2-methoxy)-ethyl-{4-N,N-bis[2-hydroxy-3-(3-methylphenoxy)-1-propyl]-amino-3-methyl-butyl}-ester.

1,4-Dihydro-2,6-dimethyl-4-(2,3-dichlorphenyl)-pyridin-3,5-dicarbonsäure-cyclopentyl-{-3-N,N-bis[2-hydroxy-3-(4-acetamidophenoxy)-1-propyl]-aminopropyl}-ester.

1,4-Dihydro-2,6-dimethyl-4-(2-nitrophenyl)-pyridin-3,5-dicarbonsäure-methyl-{2-N-[2-hydroxy-3-(1-naphthyloxy)-1-propyl]-N-[2-hydroxy-3-(4-indolyloxy)-1-propyl]-aminoethyl}-ester.

1,4-Dihydro-2,6-dimethyl-4-(3-nitrophenyl)-pyridin-3,5-dicarbonsäure-isopropyl-{4-N-[2-hydroxy-3-(4-indolyloxy)-1-propyl]-N-[2-hydroxy-3-(4-methoxyethylphenoxy)-1-propyl]-aminobutyl}-ester.

1,4-Dihydro-2,6-dimethyl-4-(3-chlorphenyl)-pyridin-3,5-dicarbonsäure-methyl-{3-N-[2-hydroxy-3-(4-aminocarbonylmethyl-phenoxy)-1-propyl]-N-[2-hydroxy-3-(1-naphthyloxy)-1-propyl]-aminopropyl}-ester.

1,4-Dihydro-2,6-dimethyl-4-(3-nitrophenyl)-pyridin-3,5-dicarbonsäure-cyclopentyl-{2-N-[2-hydroxy-3-(2-allyloxy-phenoxy)-1-propyl]-N-[2-hydroxy-3-(4-indolyloxy)-1-propyl]-aminoethyl}-ester.

1,4-Dihydro-2,6-dimethyl-4-(3-nitrophenyl)-pyridin-3,5-dicarbonsäure-(2-cyano)-ethyl-{2-[2-hydroxy-3-(4-indolyloxy)-1-propyl]-aminoethyl}-ester.

1,4-Dihydro-2,6-dimethyl-4-(2-nitrophenyl)-pyridin-3,5-dicarbonsäure-isobutyl-{5-[2-hydroxy-3-(1-naphthyloxy)-1-propyl]-aminopentyl}-ester.

1,4-Dihydro-2,6-dimethyl-4-(2-trifluormethyl-phenyl)-pyridin-3,5-dicarbonsäure-(2-methoxy)-ethyl-{4-[2-hydroxy-3-(3-methylphenoxy)-1-propyl]-amino-3-methylbutyl}-ester.

1,4-Dihydro-2,6-dimethyl-4-(2,3-dichlorphenyl)-pyridin-3,5-dicarbonsäure-cyclopentyl-{3-[2-hydroxy-3-(4-acetamidophenoxy)-1-propyl]-aminopropyl}-ester.

Die neuen erfindungsgemäßen Verbindungen haben ein breites und vielseitiges pharmakologisches Wirkungsspektrum. Sie besitzen calciumantagonistische und β-Adrenorezeptoren-blockierende Eigenschaften. Überraschenderweise hemmen sie die KCl- bzw. noradrenalininduzierten Kontraktionen von isolierten Kaninchenaorten (Versuchsanordnung : R. Towart, J. Cardiovascular Pharmacology 4, 895-902 (1982)).

Im einzelnen konnten im Tierexperiment folgende Hauptwirkungen nachgewiesen werden :

1. Die Verbindungen bewirken bei parenteraler, oraler und perlingualer Gabe eine deutliche Erweiterung der Coronargefäße. Diese Wirkung auf die Coronargefäße wird durch eine gleichzeitigen herzentlastenden Effekt verstärkt. Durch die zusätzliche antagonisierende Wirkung auf die β-adrenergen Rezeptoren hemmen die Verbindungen eine pathologisch gesteigerte Kontraktilität des Herzens. Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare antiarrhythmische Wirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z. B. dem Zentralnervensystem oder in der Niere) manifestieren. Die Verbindungen eignen sich daher besonders als Cerebraltherapeutika und als Mittel zur Behandlung von Nierenfunktionsstörungen.

4. Die Verbindungen senken den Blutdruck von normotonen und hypertonen Tieren und können somit als antihypertensive Mittel verwendet werden.

5. Die Verbindungen haven stark muskulär-spasmolytische Wirkungen, die an der glatten Muskulatur des Magens, Darmtraktes, des Urogenitaltraktes und des Respirationssystems deutlich werden.

6. Die Verbindungen senken die Herzfrequenz.

Die erfindungsgemäßen Verbindungen eignen sich aufgrund dieser Eigenschaften besonders zu Prophylaxe und Therapie der akuten und chronischen ischämischen Herzkrankheit im weitesten Sinne, zur Therapie des Hochdruks, zur Behandlung von cerebralen, renalen und systemischen Durchblutungsstörungen sowie zur Behandlung von Herzrhythmusstörungen, Schwangerschaftshochdruck, Phäochromozytom, hypertrophischen Kardiomyopathien hyperkinetischem Herzsyndrom und zur Kardioprotektion beim akuten Herzinfarkt und bei Kardioplegie als auch zur Prävention des Sekundärinfarktes.

Außerdem können die Verbindungen als Zusatzmedikation zur symptomatischen Behandlung von

Hyperthyreose, Parkinsonismus, Angstzuständen, Migräne, psychosomatischen Störungen und Glaukom angewandt werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z. B. Erdölfraktionen), pflanzliche Öle (z. B. Erdnuß-/Sesam-Öl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker), Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 5 mg/kg, vorzugsweise etwa 0,05 bis 2 mg/kg Körpergewicht pro Tag zur Erzielung wirksamer Ergebnisse zu verabreichen, und bei oraler Applikation beträgt die Dosierung etwa 0,01 bis 20 mg/kp, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht der Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Ausführungsbeispiele

### Beispiel 1

1,4-Dihydro-2,6-dimethyl-4-(3-nitrophenyl)-pyridin-3,5-dicarbonsäure-isopropyl-{2-[2-hydroxy-3-(1-naphthyloxy)-1-propyl]-aminoethyl}-ester.

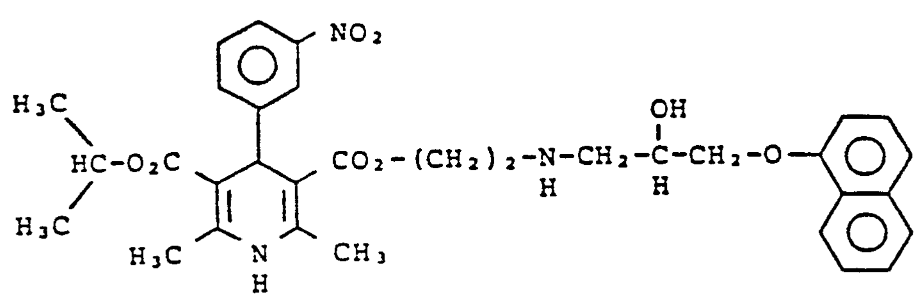

Eine Lösung von 2,0 g (5,0 mmol) 1,4-Dihydro-2,6-dimethyl-4-(3-nitrophenyl)-pyridin-3,5-dicarbonsäure-isopropyl-(2-aminoethyl)-ester in 12,5 ml Isopropanol wurde mit 1,0 g (5,0 mmol) 1-(1-Naphthyloxy)-2,3-epoxy-propan versetzt und 48 Stunden bei Raumtemperatur gerührt. Anschließend wurde im Vakuum eingeengt und der gebildete Rückstand durch zweifache Säulenchromatographie an Kieselgel (40-63 µm) mit dem Laufmittelgemisch Chloroform/Methanol/Ammoniak im Volumenverhältnis 20 : 1 : 0,05 (System 1) gereinigt. Der nach Einengen der Eluate erhaltene Kristallschaum wurde im Vakuum bei 40 °C getrocknet.

Rf : 0,33 (System 1)
Ausbeute : 1,2 g (39,8 %)

Beispiel 2

1,4-Dihydro-2,6-dimethyl-4-(3-nitrophenyl)-pyridin-3,5-dicarbonsäure-methyl-{3-[2-hydroxy-3-(1-naphthyloxy)-1-propyl]-aminopropyl}-ester.

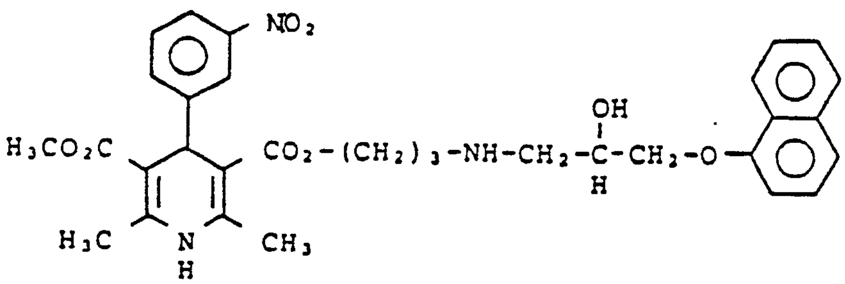

Eine Lösung von 5,9 g (15 mmol) 1,4-Dihydro-2,6-dimethyl-4-(3-nitrophenyl)-pyridin-3,5-dicarbonsäure-methyl-(3-aminopropyl)-ester in 40 ml Isopropanol wurde mit 1,5 g (7,5 mmol) 1-(1-Naphthyloxy)-2,3-epoxy-propan versetzt und 24 Stunden bei Raumtemperatur gerührt. Nach Eindampfen des Lösungsmittels unter vermindertem Druck wurde der ölige Rückstand analog Beispiel 1 säulenchromatographisch gereinigt. Der durch Einengen der Eluate erhaltene Kristallschaum wurde im Vakuum bei 40 °C getrocknet.

Rf : 0,22 (System 1)
Ausbeute : 2,47 g (55,9 %).

Beispiel 3

1,4-Dihydro-2,6-dimethyl-4-(3-nitrophenyl)-pyridin-3,5-dicarbonsäure-isopropyl-{5-[2-hydroxy-3-(1-naphthyloxy)-1-propyl]-amino-2,2-dimethyl-pentyl-}-ester.

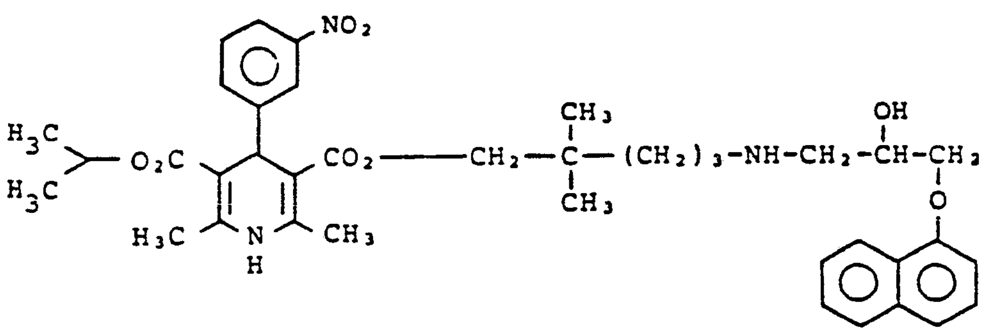

Eine Lösung von 5,7 g (12 mmol) 1,4-Dihydro-2,6-dimethyl-4-(3-nitrophenyl)-pyridin-3,5-dicarbonsäure-isopropyl-(5-amino-2,2-dimethyl-pentyl)-ester und 1,2 g (6 mmol) 1-(1-Naphthyloxy)-2,3-epoxy-propan in 42 ml Tetrahydrofuran wurde 18 Stunden auf 50 °C erhitzt. Nach Abdampfen des Lösungsmittels im Vakuum wurde der gebildete Rückstand analog Beispiel 1 säulenchromatographisch gereinigt. Der nach Einengen der Eluate erhaltene Kristallschaum wurde im Vakuum bei 40 °C getrocknet.

Rf : 0,17 (System 1)
Ausbeute : 2,54 g (62,8 %).

Beispiel 4

1,4-Dihydro-2,6-dimethyl-4-(3-nitrophenyl)-pyridin-3,5-dicarbonsäure-cyclopentyl-{3-[2-hydroxy-3-(1-naphthyloxy)-1-propyl]-aminopropyl}-ester.

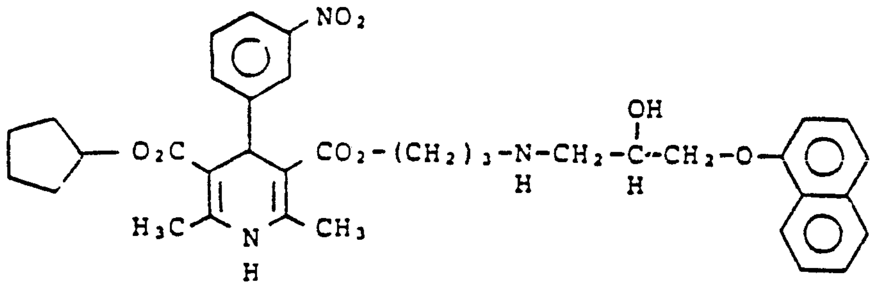

Eine Lösung von 6,1 g (13,8 mmol) 1,4-Dihydro-2,6-dimethyl-4-(3-nitrophenyl)-pyridin-3,5-dicarbonsäure-cyclopentyl-(3-aminopropyl)-ester und 1,38 g (6,9 mmol) 1-(1-Naphthyloxy)-2,3-epoxypropan in 46 ml Methylenchlorid wurde 16 Stunden unter Rückfluß erhitzt. Anschließend wurde das Lösungsmittel eingedampft und der Rückstand analog Beispiel 1 säulenchromatographisch gereinigt. Der nach Einengen der Eluate erhaltene Kristallschaum wurde bei 40 °C im Vakuum getrocknet.

Rf : 0,23 (System 1)

Ausbeute :, 2,04 g (45,9 %).

Die weiteren in der nachfolgenden Tabelle aufgeführten Beispiele wurden analog den vorstehend beschriebenen Verfahren erhalten.

(Siehe Tabelle 1 Seite 11 ff.)

Tabelle 1

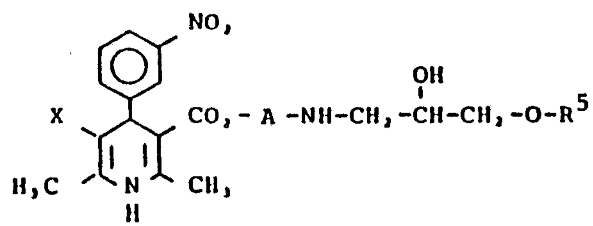

| Beispiel | X | A | $R^5$ | Reaktions- Zeit (h) | Temp. (°C) | Lösungsmittel | $R_f$ * | Ausb. (% d.Th.) |
|---|---|---|---|---|---|---|---|---|
| 5 | $H_3COOC$ | $-(CH_2)_2-$ | α-Naphthyl | 48 | 22 | Isoprop. | 0,19 | 50,0 |
| 6 | $H_3COOC$ | $-(CH_2)_6-$ | α-Naphthyl | 40 | 23 | Isoprop. | 0,18 | 26,1 |
| 7 | $(H_3C)_2CHOOC$ | $-(CH_2)_3-$ | α-Naphthyl | 40 | 22 | Isoprop. | 0,20 | 59,2 |
| 8 | $(H_3C)_3COOC$ | $-(CH_2)_2-$ | α-Naphthyl | 18 | 50 | THF | 0,23 | 42,5 |
| 9 | $(H_3C)_2CHOOC$ | $-(CH_2)_5-$ | α-Naphthyl | 24 | 22 | Isoprop. | 0,18 | 65,7 |
| 10 | $(H_3C)_3COOC$ | $-(CH_2)_3-$ | α-Naphthyl | 15 | 40 | $CH_2Cl_2$ | 0,14 | 52,9 |
| 11 | $H_3COOC$ | 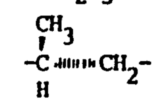 | α-Naphthyl | 48 | 22 | Isoprop. | 0,22 | 74,8 |
| 12 | $H_3COOC$ | 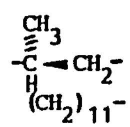 | α-Naphthyl | 48 | 22 | Isoprop. | 0,25 | 69,9 |
| 13 | $(H_3C)_2CHOOC$ | $(CH_2)_{11}-$ | α-Naphthyl | 20 | 50 | THF | 0,19 | 45,6 |
| 14 | $H_3COOC$ | $-(CH_2)_{11}-$ | α-Naphthyl | 20 | 50 | THF | 0,14 | 47,5 |
| 15 | $H_3COOC$ | $-(CH_2)_4-$ | α-Naphthyl | 16 | 40 | $CH_2Cl_2$ | 0,14 | 54,8 |
| 16 | $H_3COOC$ | $-(CH_2)_5-$ | α-Naphthyl | 16 | 40 | $CH_2Cl_2$ | 0,16 | 62,3 |

* Als Elutionsmittel verwendete man das Gemisch $CHCl_3/MeOH/NH_3$ im Volumenverhältnis 20 : 1 : 0,05

| Beispiel | X | A | $R^5$ | Reaktions-Zeit (h) | Temp. (°C) | Lösungsmittel | $R_f$* | Ausb. (% d.Th.) |
|---|---|---|---|---|---|---|---|---|
| 17 | $(H_3C)_2CHOOC-$ | $-(CH_2)_6-$ | α-Naphthyl | 48 | 40 | $CH_2Cl_2$ | 0.20 | 35.9 |
| 18 | $(H_3C)_3COOC-$ | $-(CH_2)_5-$ | α-Naphthyl | 24 | 40 | $CH_2Cl_2$ | 0.16 | 54.1 |
| 19 | $(H_3C)_2CHOOC-$ | $-(CH_2)_4-$ | α-Naphthyl | 36 | 20 | Isoprop. | 0.19 | 58.7 |
| 20 | $H_3COOC-$ | $-(CH_2)_2-O-(CH_2)_2-$ | α-Naphthyl | 36 | 20 | Isoprop. | 0.17 | 65.3 |
| 21 | $H_3COOC-$ | 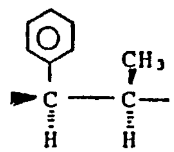 | α-Naphthyl | 48 | 20 | Isoprop. | 0.30 | 66.8 |
| 22 | $(H_3C)_3C-CH_2OOC-$ | $(H_2C)_3-CH(CH_3)-$ | α-Naphthyl | 48 | 20 | Isoprop. | 0.18 | 35.3 |
| 23 | $(H_3C)_2CHOOC-$ | $(H_2C)_3-CH(CH_3)-$ | α-Naphthyl | 24 | 82 | Isoprop. | 0.15 | 44.8 |
| 24 | $H_3COOC-$ | 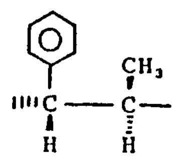 | α-Naphthyl | 48 | 82 | Isoprop. | 0.35 | 38.9 |

* Als Elutionsmittel verwendete man das Gemisch $CHCl_3/MeOH/NH_3$ im Volumenverhältnis 20 : 1 : 0.05

| Beispiel | X | A | $R^5$ | Reaktions-Zeit (h) | Temp. (°C) | Lösungsmittel | $R_f$ * | Ausbeute (% d. Th.) |
|---|---|---|---|---|---|---|---|---|
| 25 | $H_3COOC-$ | $-(CH_2)_2-$ | 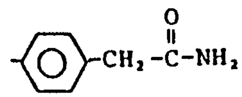 | 72 | 20 | THF | 0.06 | 16.2 |
| 26 | $(H_3C)_2CHOOC-$ | $-(CH_2)_3-$ | 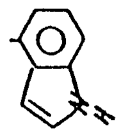 | 7 | 82 | Isoprop. | 0.13 | 72.6 |
| 27 | $(H_3C)_2CHOOC-$ | 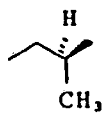 | α-Naphthyl | 48 | 20 | Isoprop. | 0.27 | 48.6 |
| 28 | $(H_3C)_2CHOOC-$ | 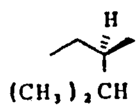 | α-Naphthyl | 24<br>24 | 20<br>82 | Isoprop. | 0.37 | 90.0 |
| 29 | $(H_3C)_2CHOOC-$ | 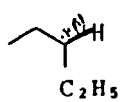 | α-Naphthyl | 24 | 82 | Isoprop. | 0.35 | 48.8 |

* Als Elutionsmittel verwendete man das Gemisch $CHCl_3/MeOH/NH_3$ im Volumenverhältnis 20 : 1 : 0.05

| Beispiel | X | A | $R^5$ | Reaktions Zeit (h) | /Temp. (°C) | Lösungsmittel | $R_f$ * | Ausb. (% d.Th.) |
|---|---|---|---|---|---|---|---|---|
| 30 | $(H_3C)_2CHOOC-$ | $-(CH_2)_2-O-(CH_2)_2$ | α-Naphthyl | 24 | 82 | Isoprop. | 0.19 | 78.9 |
| 31 | $(H_3C)_2CHOOC-$ | $-(CH_2)_2-S-(CH_2)_2$ | α-Naphthyl | 48 | 82 | Isoprop. | 0.28 | 73.2 |
| 32 | $H_3COOC-$ | $-(CH_2)_2-N(CH_3)-(CH_2)_3-$ | α-Naphthyl | 48 | 82 | Isoprop. | 0.06 | 51.0 |

* Als Elutionsmittel verwendete man das Gemisch $CHCl_3/MeOH/NH_3$ im Volumenverhältnis 20 : 1 : 0.05

Beispiel 33

1,4-Dihydro-2,5-dimethyl-4-(3-nitrophenyl)-pyridin-3,5-dicarbonsäure-isopropyl-{3-N,N-bis-[2-hydroxy-3-(1-naphthyloxy)-1-propyl]-aminopropyl}-ester.

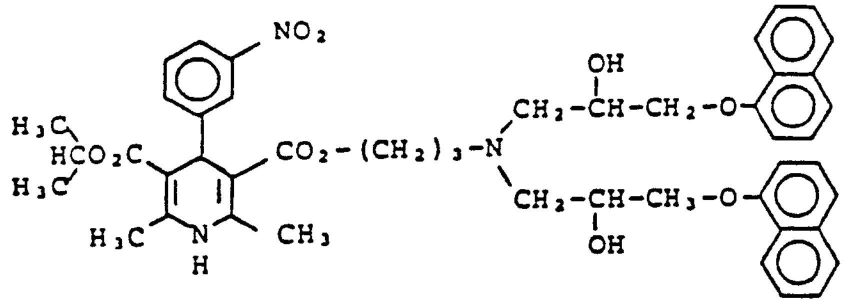

Eine Lösung von 1,7 g (4 mmol) 1,4-Dihydro-2,6-dimethyl-4-(3-nitrophenyl)-pyridin-3,5-dicarbonsäure-isopropyl-(3-aminopropyl)-ester und 2,4 g (12 mmol) 1-(1-Naphthyloxy)-2,3-epoxy-propan in 12 ml Isopropanol wurde 48 Stunden bei Raumtemperatur gerührt. Anschließend wurde im Wakuum eingeengt und der gebildete Rückstand durch Säulenchromatographie an Kieselgel (40-63 µm) mit dem Laufmittelgemisch Chloroform/Methanol/Ammoniak im Volumenverhältnis 20 : 1 : 0,05, (System 1) gereinigt. Der nach Einengen der Eluate erhaltene Kristallschaum wurde im Vakuum bei 40 °C getrocknet.

Rf : 0,39 (System 1)

Ausbeute : 2,3 g (70,3 %)

Beispiel 34

1,4-Dihydro-2,5-dimethyl-4-(3-nitrophenyl)-pyridin-3,5-dicarbonsäure-methyl-{2-N,N-bis[2-hydroxy-3-(1-naphthyloxy)-1-propyl]-aminoethyl}-ester.

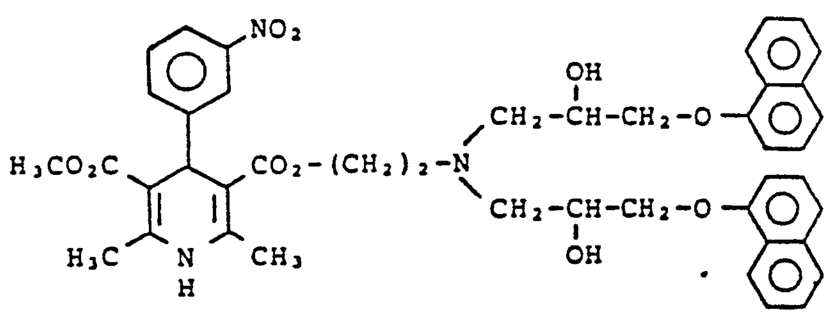

Eine Lösung von 1,9 g (5 mmol) 1,4-Dihydro-2,6-dimethyl-4-(3-nitrophenyl)-pyridin-3,5-dicarbonsäure-methyl-(2-aminoethyl)-ester und 2,0 g (10 mmol) 1-(1-Naphthyloxy)-2,3-epoxy-propan in 14 ml Isopropanol wurde 16 Stunden zum Sieden erhitzt. Anschließend wurde im Vakuum eingeengt und der gebildete Rückstand durch Säulenchromatographie an Kieselgel analog Beispiel 17 gereinigt. Der durch Einengen der Eluate erhaltene Kristallschaum wurde im Vakuum bei 40 °C getrocknet.

Rf : 0,32 (System 1).

Ausbeute : 2,66 g (68,6 %).

Beispiel 35

1,4-Dihydro-2,6-dimethyl-4-(3-nitrophenyl)-pyridin-3,5-dicarbonsäure-cyclopentyl-{3-N,N-bis[2-hydroxy-3-(1-naphthyloxy)-1-propyl]-aminopropyl}-ester.

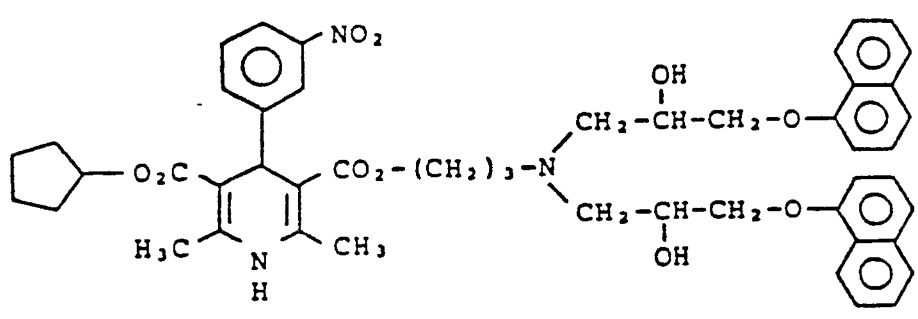

Eine Lösung von 3,06 g (6,9 mmol) 1,4-Dihydro-2,6-dimethyl-4-(3-nitrophenyl)-pyridin-dicarbonsäure-cyclopentyl-(3-aminopropyl)-ester und 2,76 g (13,8 mmol) 1-(1-Naphthyloxy)-2,3-epoxypropan in 20 ml Methylenchlorid wurde 20 Stunden zum Sieden erhitzt. Anschließend wurde das Lösungsmittel unter vermindertem Druck abdestilliert und der Rückstand analog Beispiel 17 säulenchromatographisch gereinigt. Der nach Einengen der Eluate erhaltene Kristallschaum wurde bei 40 °C im Vakuum getrocknet.

Rf : 0,42 (System 1)

Ausbeute : 3,83 g (65,7 %).

Beispiel 36

1,4-Dihydro-2,6-dimethyl-4-(3-nitrophenyl)-pyridin-3,5-dicarbonsäure-methyl-{11-N,N-bis[2-hydroxy-3-(1-naphthyloxy)-1-propyl]-aminoundecyl}-ester.

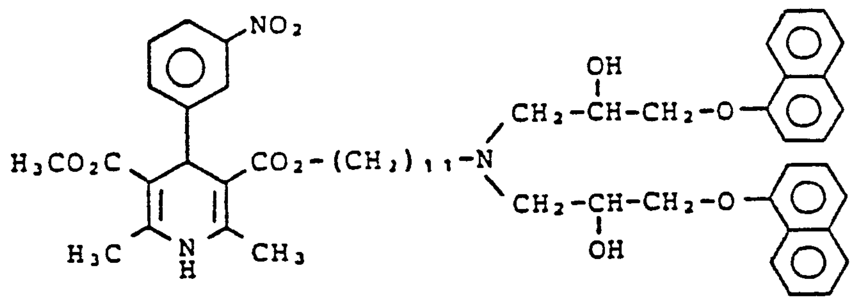

Eine Lösung von 2,71 g (4,3 mmol) 1,4-Dihydro-2,6-dimethyl-4-(3-nitrophenyl)-pyridin-3,5-dicarbonsäure-methyl-(11-aminoundecyl)-ester und 1,72 g (8,6 mmol) 1-(1-Naphthyloxy)-2,3-epoxy-propan in 21 ml Tetrahydrofuran wurde 20 Stunden auf 50 °C erhitzt. Nach Abdampfen des Lösungsmittels im Vakuum wurde der gebildete Rückstand analog Beispiel 17 säulenchromatographisch gereinigt. Der nach Einengen der Eluate erhaltene Kristallschaum wurde im Vakuum bei 40 °C getrocknet.

Rf : 0,45 (System 1)

Ausbeute : 2,18 g (56,3 %).

Die weiteren in der nachfolgenden Tabelle aufgeführten Beispiele wurden analog den vorstehend beschriebenen Verfahren erhalten.

(Siehe Tabelle 2 Seite 17 ff.)

Tabelle 2

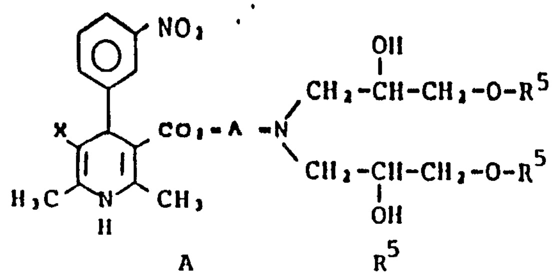

| Beispiel | X | A | $R^5$ | Reaktions-Zeit (h) | Reaktions-Temp. (°C) | Lösungsmittel | Rf* | Ausbeute (% d.Th.) |
|---|---|---|---|---|---|---|---|---|
| 37 | $(H_3C)_2CHOOC$ | $-(CH_2)_2-$ | α-Naphthyl | 48 | 22 | Isoprop. | 0,43 | 72,3 |
| 38 | $H_3COOC$ | $-(CH_2)_3-$ | α-Naphthyl | 16 | 82 | Isoprop. | 0,35 | 66,8 |
| 39 | $H_3COOC$ | $-(CH_2)_6-$ | α-Naphthyl | 20 | 40 | $CH_2Cl_2$ | 0,31 | 51,6 |
| 40 | $(H_3C)_3COOC$ | $-(CH_2)_2-$ | α-Naphthyl | 24 | 82 | Isoprop. | 0,41 | 63,2 |
| 41 | $(H_3C)_2CHOOC$ | 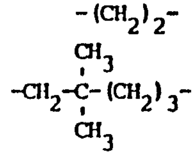 | α-Naphthyl | 16 | 50 | THF | 0,36 | 57,8 |
| 42 | $(H_3C)_2CHOOC$ | $-(CH_2)_5-$ | α-Naphthyl | 20 | 40 | $CH_2Cl_2$ | 0,45 | 65,0 |
| 43 | $(H_3C)_2CHOOC$ | $-(CH_2)_{11}-$ | α-Naphthyl | 22 | 40 | $CH_2Cl_2$ | 0,53 | 62,5 |
| 44 | $(H_3C)_3COOC$ | $-(CH_2)_3-$ | α-Naphthyl | 40 | 22 | Isoprop. | 0,44 | 71,9 |
| 45 | $H_3COOC$ | 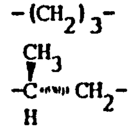 | α-Naphthyl | 40 | 22 | Isoprop. | 0,41 | 74,2 |
| 46 | $H_3COOC$ | 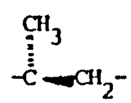 | α-Naphthyl | 40 | 22 | Isoprop. | 0,43 | 76,0 |
| 47 | $H_3COOC$ | $-(CH_2)_4-$ | α-Naphthyl | 20 | 40 | $CH_2Cl_2$ | 0,37 | 68,3 |
| 48 | $H_3COOC$ | $-(CH_2)_5-$ | α-Naphthyl | 22 | 40 | $CH_2Cl_2$ | 0,42 | 70,8 |

* Als Elutionsmittel verwendete man das Gemisch $CHCl_3/MeOH/NH_3$ im Volumenhältnis 20 : 1 : 0,05

| Beispiel | X | A | $R^5$ | Reaktions-Zeit (h) | Reaktions-Temp. (°C) | Lösungsmittel | $R_f$* | Ausb. (% d.Th.) |
|---|---|---|---|---|---|---|---|---|
| 49 | $(H_3C)_2CHOOC$ | $-(CH_2)_6-$ | α-Naphthyl | 48 | 40 | $CH_2Cl_2$ | 0.42 | 57.2 |
| 50 | $(H_3C)_3COOC$ | $-(CH_2)_5-$ | α-Naphthyl | 24 | 40 | $CH_2Cl_2$ | 0.35 | 65.9 |
| 51 | $(H_3C)_2CHOOC$ | $-(CH_2)_4-$ | α-Naphthyl | 36 | 20 | Isoprop. | 0.40 | 66.3 |
| 52 | $H_3COOC$ | $-(CH_2)_2-O-(CH_2)_2-$ | α-Naphthyl | 36 | 20 | Isoprop. | 0.49 | 68.1 |
| 53 | $H_3COOC$ | 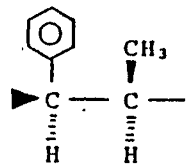 | α-Naphthyl | 48 | 82 | Isoprop. | 0.58 | 67.7 |
| 54 | $(H_3C)_3C-CH_2OOC$ | $-(CH_2)_3-CH-$<br>$CH_3$ | α-Naphthyl | 48 | 82 | Isoprop. | 0.48 | 60.1 |
| 55 | $(H_3C)_2CHOOC$ | $-(CH_2)_3-CH-$<br>$CH_3$ | α-Naphthyl | 24 | 82 | Isoprop. | 0.43 | 62.3 |
| 56 | $(H_3C)_2CHOOC$ | $-(CH_2)_3$ | 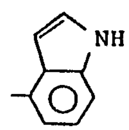 | 7 | 82 | Isoprop. | 0.20 | 65.4 |

* Als Elutionsmittel verwendete man das Gemisch $CHCl_3/MeOH/NH_3$ im Volumen 20 : 1 . 0.05

| Beispiel | X | A | $R^5$ | Reaktions-Zeit (h) | /Temp. (°C) | Lösungsmittel | $R_f$* | Ausb. (%d.Th.) |
|---|---|---|---|---|---|---|---|---|
| 57 | $(H_3C)_2CHOOC$ | 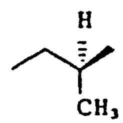 | α-Naphthyl | 24 | 82 | Isoprop. | 0.46 | 55.8 |
| 58 | $(H_3C)_2CHOOC$ | 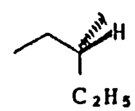 | α-Naphthyl | 72 | 82 | Isoprop. | 0.62 | 62.3 |
| 59 | $(H_3C)_2CHOOC$ | $-(CH_2)_2-O-(CH_2)_2-$ | α-Naphthyl | 24 | 82 | Isoprop. | 0.41 | 70.5 |
| 60 | $(H_3C)_2CHOOC$ | $-(CH_2)_2-S-(CH_2)_2-$ | α-Naphthyl | 24 | 82 | Isoprop. | 0.42 | 65.6 |
| 61 | $H_3COOC$ | $-(CH_2)_2-N(CH_3)-(CH_2)_3$ | α-Naphthyl | 48 | 82 | Isoprop. | 0.25 | 60.3 |

* Als Elutionsmittel verwendete man das Gemisch $CHCl_3/MeOH/NH_3$ im Volumenverhältnis 20 : 1 : 0.05

Beispiel 62

1,4-Dihydro-2,6-dimethyl-4-(3-nitrophenyl)-pyridin-3,5-dicarbonsäure-methyl-{2-N-[2-hydroxy-3-(1-naphthyloxy)-1-propyl]-N-methyl-aminoethyl}-ester.

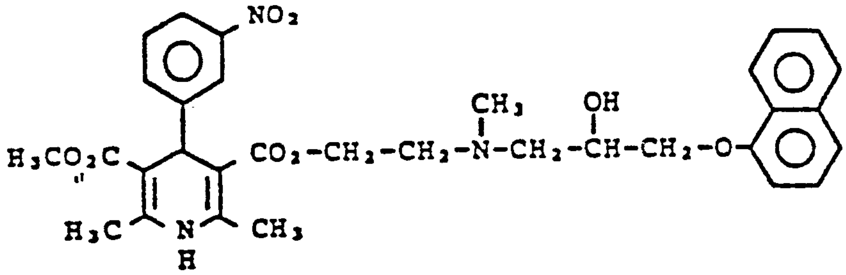

Eine Lösung von 1,95 g (5 mmol) 1,4-Dihydro-2,6-dimethyl-4-(3-nitrophenyl)-pyridin-3,5-dicarbonsäure-methyl-(2-N-methylaminoethyl)-ester in 30 ml Isopropanol wurde mit 1,1 g (5,5 mmol) 1-(1-Naphthyloxy)-2,3-epoxy-propan versetzt und 48 Stunden bei Raumtemperatur gerührt. Nach Eindampfen des Lösungsmittels unter vermindertem Druck wurde der ölige Rückstand analog Beispiel 1 säulenchromatographisch gereinigt. Der durch Einengen der Eluate erhaltene Kristallschaum wurde im Vakuum bei 40 °C getrocknet.

RF : 0,38 (System 1).

Ausbeute : 2,13 g (72,2 %).

Beispiel 63

1,4-Dihydro-2,6-dimethyl-4-(3-nitrophenyl)-pyridin-3,5-dicarbonsäure-isopropyl-{11-N-[2-hydroxy-3-(4-aminocarbonylmethyl-phenoxy)-1-propyl]-N-[2-hydroxy-3-(1-naphthyloxy)-1-propyl]-aminoundecyl}-ester.

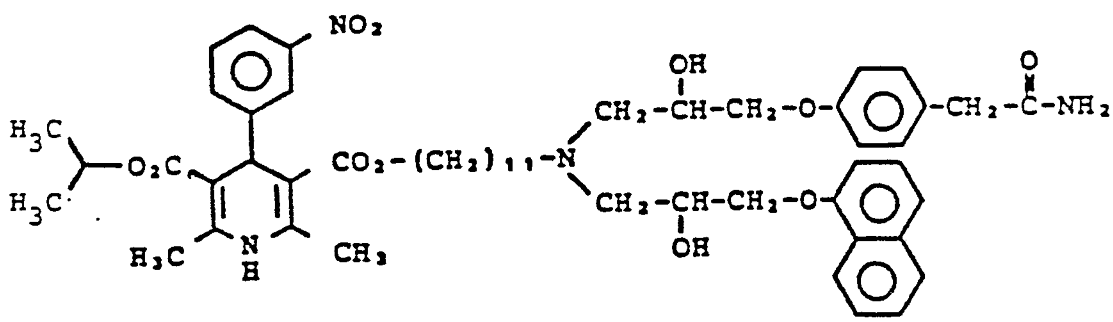

Eine Lösung von 1,0 g (1,37 mmol) 1,4-Dihydro-2,6-dimethyl-4-(3-nitrophenyl)-pyridin-3,5-dicarbonsäure-isopropyl-{11-[2-hydroxy-3-(1-naphthyloxy-1-propyl]-aminoundecyl}-ester und 0,57 g (2,74 mmol) 1-(4-Aminocarbonyl-methyl-phenoxy)-2,3-epoxy-propan in 20 ml Isopropanol wurde 18 Stunden zum Sieden erhitzt. Anschließend wurde im Vakuum eingeengt und der gebildete Rückstand durch Säulenchromatographie an Kieselgel analog Beispiel 1 gereinigt. Der durch Einengen der Eluate erhaltene Kristallschaum wurde im Vakuum bei 40 °C getrocknet.

Rf : 0:05 (System 1).

Ausbeute : 0,62 g (48,8 %).

Beispiel 64

1,4-Dihydro-2,6-dimethyl-4-(3-nitrophenyl)-pyridin-3,5-dicarbonsäure-methyl-{2-N-[2-hydroxy-3-(1-naphthyloxy)-1-propyl]-N-methyl-carbonyl-aminoethyl}-ester.

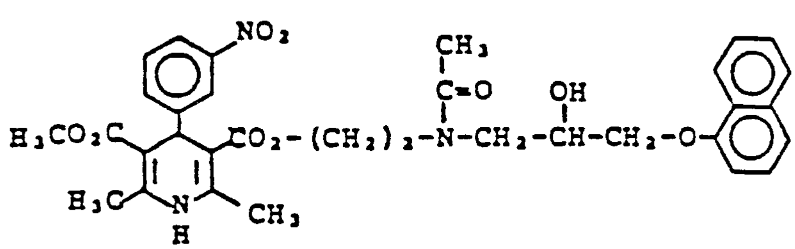

Eine lösung von 1,73 g (3,0 mmol) 1,4-Dihydro-2,6-dimethyl-4-(3-nitrophenyl)-pyridin-3,5-dicarbonsäure-methyl-{2-[2-hydroxy-3-(1-naphthyloxy)-1-propyl]-aminoethyl}-ester und 332 mg (3,3 mmol) Triethylamin in 20 ml Dioxan wurde tropfenweise mit 337 g (1,1 mmol) Acetanhydrid gelöst in 2 ml Dioxan versetzt und nach Beendigung der Zugabe noch 3 Stunden bei Raumtemperatur gerührt. Nach Einengen der Dioxanlösung wurde das Reaktionsgemisch in Methylenchlorid aufgenommen, nacheinander mit verdünnter Salzsäure, Hydrogencarbonatlösung und Wasser gewaschen und die Methylenchloridlösung mit Natriumsulfat getrocknet. Nach Abdampfen der organischen Phase wurde der gebildete Rückstand säulenchromatographisch analog Beispiel 17 gereinigt.

Ausbeute : 0,9 g (48,6 %).

Rf : 0.37 (System 1)

Beispiel 65

1,4-Dihydro-2,6-dimethyl-4-(3-nitrophenyl)-pyridin-3,5-dicarbonsäure-isopropyl-{3-N-ethoxycarbonyl-N-[2-hydroxy-3-(1-naphthyloxy)-1-propyl]-aminopropyl}-ester.

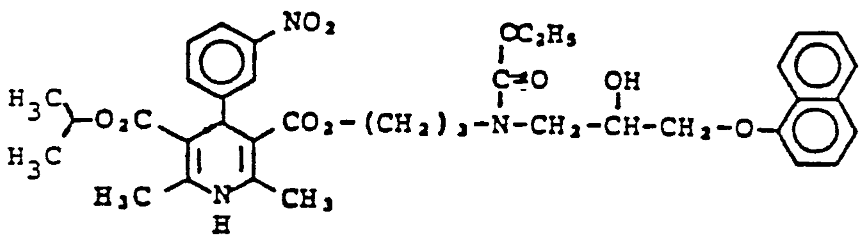

2,2 g (3,6 mmol) 1,4-Dihydro-2,6-dimethyl-4-(3-nitrophenyl)-pyridin-3,5-dicarbonsäure-isopropyl-{3-[2-hydroxy-3-(1-naphthyloxy)-1-propyl]-aminopropyl}-ester wurden zusammen mit 400 mg (4,0 mmol) Triethylamin in 20 ml Methylenchlorid gelöst und bei 10 °C tropfenweise mit 391 mg (3,6 mmol) Chlorkohlensäureethyl-ester gelöst in 2 ml Methylenchlorid versetzt. Nach Beendigung der Zugabe wurde noch 1 Stunden bei Raumtemperatur gerührt und anschließend analog Beispiel 35 aufgearbeitet.

Ausbeute : 1,74 g (70,1 %)

Rf : 0.21 ($CH_2Cl_2$/EtoAC = 5/1).

Beispiel 66

1,4-Dihydro-2,6-dimethyl-4-(3-nitrophenyl)-pyridin-3,5-dicarbonsäure-methyl-{2-N-[2-hydroxy-3-(1-naphthyloxy)-1-propyl]-N-phenylamino-carbonyl-aminoethyl}-ester.

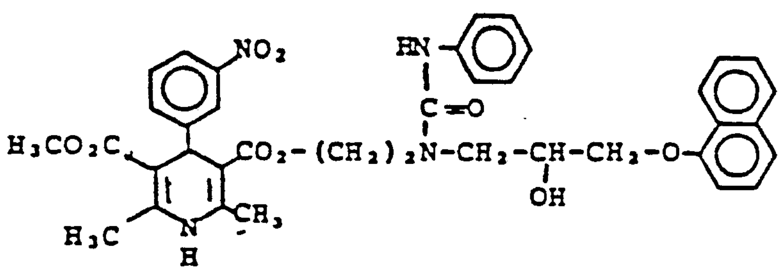

Eine Lösung von 2,3 g (4,0 mmol) 1,4-Dihydro-2,6-dimethyl-4-(3-nitrophenyl)-pyridin-3,5-dicarbonsäure-methyl-{2-[2-hydroxy-3-(1-naphthyloxy)-1-propyl]-aminoethyl}-ester in 30 ml Methylenchlorid wurde bei 10 °C tropfenweise mit 477 mg (4,0 mmol) Phenylisocyanat gelöst in 5 ml Methylenchlorid versetzt. Nach Beendigung der Zugabe wurde noch 1 Stunde bei Raumtemperatur gerührt, und analog Beispiel 35 aufgearbeitet.

Ausbeute : 2,25 g (81,0 %)

Rf : 0.29 ($CH_2Cl_2$/EtoAC = 5/1).

Beispiel 67

1,4-Dihydro-2,6-dimethyl-4-(3-nitrophenyl)-pyridin-3,5-dicarbonsäure-isopropyl-{2-N-[2-hydroxy-3-(1-naphthyloxy)-1-propyl]-N-tosyl-aminopropyl}-ester.

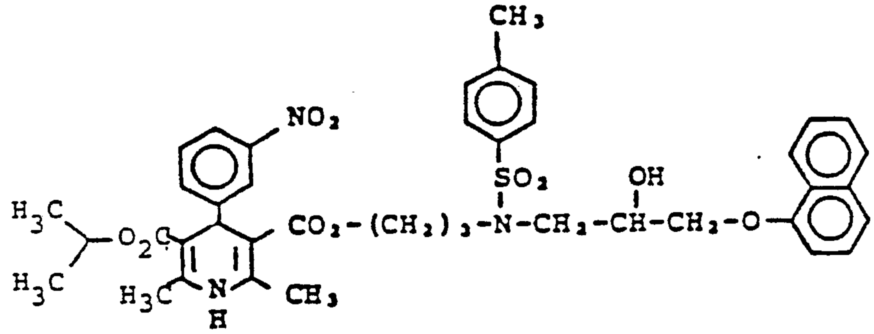

2,5 g (4,0 mmol) 1,4-Dihydro-2,6-dimethyl-4-(3-nitrophenyl)-pyridin-3,5-dicarbonsäure-isopropyl-{3-[2-hydroxy-3-(1-naphthyloxy)-1-propyl]-aminopropyl}-ester und 350 mg (4,4 mmol) Pyridin wurden in 15 ml Methylenchlorid gelöst und bei 10 °C tropfenweise mit einer Lösung von 839 mg (4,4 mmol) Toluolsulfonylchlorid gelöst in 5 ml Methylenchlorid versetzt. Nach Beendigung der Zugabe wurde noch 2 Stunden bei Raumtemperatur gerührt und anschließend analog Beispiel 35 aufgearbeitet.

Ausbeute : 2,1 g (68,3 %)

Rf : 0.44 ($CH_2Cl_2$/EtoAC = 5/1).

Tabelle 3

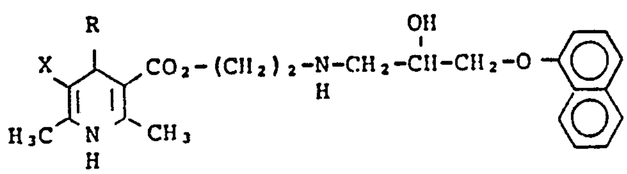

| Beispiel | X | R | Reaktions- Zeit (h)/ | Temp. (°C) | Lösungs- mittel | $R_f$* | Ausb. (% d. Th.) |
|---|---|---|---|---|---|---|---|
| 68 | $H_3COOC-$ | 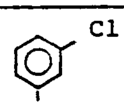 | 24 | 82 | Isoprop. | 0.30 | 76.3 |
| 69 | $H_3COOC-$ | 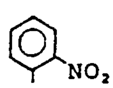 | 48 | 82 | Isoprop. | 0.22 | 58.9 |
| 70 | $H_5C_2OOC-$ | 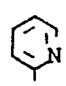 | 24 | 82 | Isoprop. | 0.08 | 45.6 |
| 71 | $(H_3C)_2CHOOC-$ | 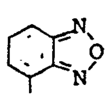 | 24 | 82 | Isoprop. | 0.12 | 73.8 |

* Als Elutionsmittel verwendet man das Gemisch $CHCl_3$/MeOH/$NH_3$ im Volumenverhältnis 20 : 1 : 0.05.

Die erfindungsgemäßen neuen Verbindungen werden Ratten mit einer Dosis von 1 mg/kg intravenös appliziert und der Blutdruck vor und nach der Applikation bestimmt.

In einem zweiten Test wird die Herzfrequenz von Ratten durch subkutane Applikation von Isoprenalin stimuliert. Dann werden die erfindungsgemäßen Verbindungen appliziert (1 mg/kg i.v.) und der Effekt auf die Erniedrigung der Herzfrequenz gemessen.

Tabelle 4 enthält die Ergebnisse dieser Tests für eine repräsentative Zahl der erfindungsgemäßen Verbindung.

Tabelle 4

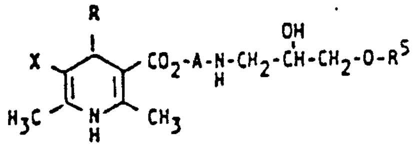

| Beispiel | X | - R | A | $R^5$ | anästhesierte Ratten 1 mg/kg i.v. BD* | HR** |
|---|---|---|---|---|---|---|
| 1 | $(H_3C)_2CHOOC$- | 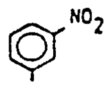 | $-(CH_2)_2$ | 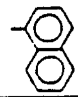 | ↓↓ | ↓↓ |
| 11 | $H_3COOC$ | 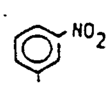 | $-\overset{CH_3}{C}-CH_2-$ | 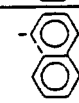 | ↓ | ↓↓ |
| 68 | $H_3COOC$ | 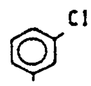 | $-(CH_2)_2-$ | 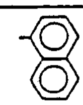 | ↓↓ | ↓ |
| 69 | $H_3COOC$ | 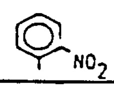 | $-(CH_2)_2-$ | 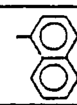 | ↓ | ↓ |
| 16 | $H_3COOC$ | 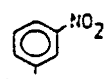 | $-(CH_2)_5-$ | 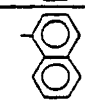 | ↓ | ↓ |
| 20 | $H_3COOC$ | 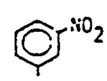 | $-(CH_2)_2-O-(CH_2)_2$ | 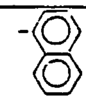 | ↓ | ↓↓ |
| 30 | $(H_3C)_2CHOOC$- | 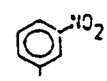 | $-(CH_2)_2-O-(CH_2)_2$ | 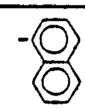 | ↓↓↓ | (↓) |
| 31 | $(H_3C)_2CHOOC$- | 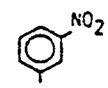 | $-(CH_2)_2-S-(CH_2)_2$ | 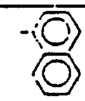 | ↓↓ | ↓↓ |
| 32 | $H_3COOC$- | 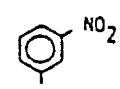 | $-(CH_2)_2-\overset{CH_3}{N}-(CH_2)_3$ | 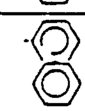 | ↓ | ↓ |
| 25 | $H_3COOC$- | 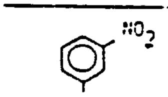 | $-(CH_2)_2-$ | 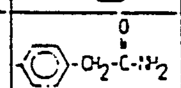 | (↓) | ↓ |
| 26 | $(H_3C)_2CHOOC$- | 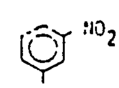 | $-(CH_2)_3-$ | 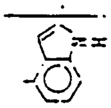 | ↓ | ↓↓↓ |
| 29 | $(H_3C)_2CHOOC$- | 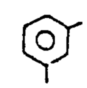 | 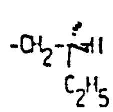 | 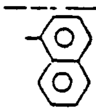 | ↓↓↓ | ↓↓↓ |

* BD : Blutdrucksenkung in % bei normotonen Ratten

(↓) 5 - < 10
↓ 10 - < 20
↓↓ 20 - < 30
↓↓↓ 30 - < 40

** HR : Erniedrigung der Herzfrequenz in absoluten Zahlen bei Isoprenalin stimulierten Ratten (subkutane Applikation)

(↓) 10
↓ 15 - < 40
↓↓ 40 < 65
↓↓↓ 65 < 100

**Patentansprüche**

1. Verbindungen der allgemeinen Formel (I)

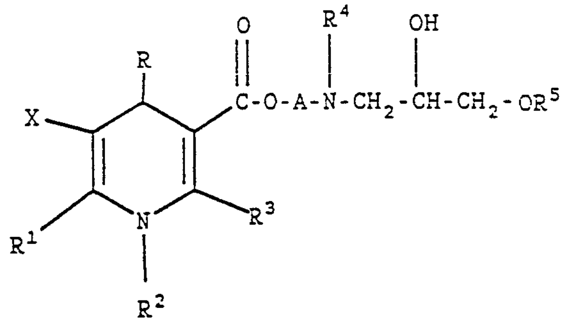

(I)

in welcher

R für Phenyl, Pyridyl oder den Benzoxadiazolylrest steht, wobei der Phenylrest 1 oder 2 gleiche oder verschiedene Substituenten aus der Gruppe Nitro, Trifluormethyl, Fluor, Chlor oder Cyano enthält,

$R^1$ und $R^3$ gleich oder verschieden sind und jeweils für Alkyl mit 1 bis 4 Kohlenstoffatomen stehen oder einer der Substituenten $R^1$ oder $R^3$ für Hydroxymethyl, Amino oder Cyano steht,

$R^2$ für Wasserstoff oder Alkyl mit 1 bis 2 Kohlenstoffatomen steht,

A entweder für eine durch Methyl, Ethyl oder Propyl substituerte Alkylengruppe mit bis zu 12 Kohlenstoffatomen steht, die gegebenenfalls durch eine Phenylgruppe substituiert oder unterbrochen ist und/oder die gegebenenfalls durch ein Sauerstoff- oder Schwefelatom oder eine Gruppierung der Formel $—NR^6$ unterbrochen ist,

oder

A für eine geradkettige oder verzweigte Alkylengruppe mit bis zu 12 Kohlenstoffatomen steht, die gegebenenfalls durch 1 Sauerstoff- oder Schwefelatom oder eine Gruppierung der Formel $—NR^6$ unterbrochen ist, wenn

a) $R^4$ für Phenyl, Benzyl, $CO—R^6$, $CO_2—R^6$, $CO—NR^6R^7$, $SO_2R^6$ oder $—CH_2—CHOH—CH_2—OR^8$ steht, oder

b) R für Pyridyl oder Benzoxadiazolyl steht, oder

c) $R^5$ für Indolyl steht, oder

d) die Alkylenkette durch ein Schwefelatom oder die Gruppe $—NR^6$ unterbrochen ist oder

e) X für $COR^9$ steht,

wobei

$R^6$ und $R^7$ gleich oder verschieden sind und jeweils für Wasserstoff, Alkyl mit bis zu 4 C-Atomen, Benzyl oder Phenyl stehen und wobei

$R^8$ die für $R^5$ angegebene Bedeutung hat und mit diesen gleich oder verschieden sein kann und wobei

$R^9$ für Alkyl mit 1 bis 7 C-Atomen steht, welches gegebenenfalls durch Halogen, Cyano, Alkoxy oder Dialkylamino mit jeweils 1-4 C-Atomen substituiert ist, oder Phenyl, Naphthyl, Phenylalkyl oder Anilino oder eine Amino-, Monoalkylamino- oder Dialkylaminogruppe mit je 1 bis 4 C-Atomen bedeutet, wobei gegebenenfalls die Alkylgruppen mit dem Stickstoffatom einen 5- bis 7-gliedrigen Ring bilden, der als weiteres Heteroatom ein Sauerstoffatom enthalten kann und wobei

$R_6{}'$ die Bedeutung von $R^6$ hat jedoch nicht Wasserstoff bedeutet,

$R^4$ für Wasserstoff, für geradkettiges oder verzweigtes Alkyl mit 1 bis 3 Kohlenstoffatomen, für Phenyl, Benzyl oder für einen Rest der Formel $CO—R^6$, $CO_2$-$R^{6'}$, $CO—NR^6R^7$, $SO_2R^6$ oder $—CH_2—CHOH—CH_2—OR^8$ steht, wobei

$R^6$, $R^{6'}$, $R^7$, $R^8$ und $R^9$ die oben angegebene Bedeutung haben,

$R^5$ für Phenyl, Naphthyl oder Indolyl steht, wobei der Phenylrest gegebenenfalls durch 1 oder 2 gleiche oder verschiedene Substituenten aus der Gruppe Fluor, Chlor, Nitro, Cyano, Alkyl mit 1 bis 2 C-Atomen, $NR^6$, $R^7$, $NR^6COR^7$, die Gruppe $CH_2—CO—NH_2$ oder Acetyl substituiert ist und

X für die Gruppe $COOR^{10}$ steht, wobei $R^{10}$ für einen geradkettigen, verzweigten oder cyclischen Alkylrest mit bis zu 12 C-Atomen steht, der gegebenenfalls durch ein Sauerstoffatom in der Kette unterbrochen ist und/oder der gegebenenfalls substituiert ist durch Fluor, Chlor, Hydroxy, Pyridyl, Phenyl oder Amino oder für die Gruppe $COR^9$ steht, worin $R^9$ die oben angegebene Bedeutung hat,

sowie deren pharmazeutisch unbedenklichen Säureadditionssalze.

2. Verbindung der Formel

(Siehe Formel Seite 25 f.)

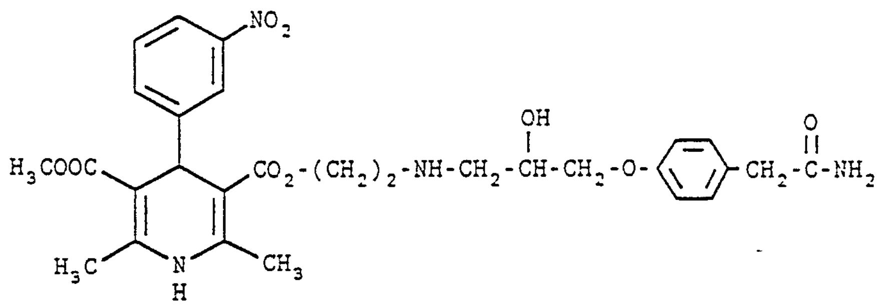

3. Verfahren zur Herstellung von Verbindungen der allgemeinen Formel I

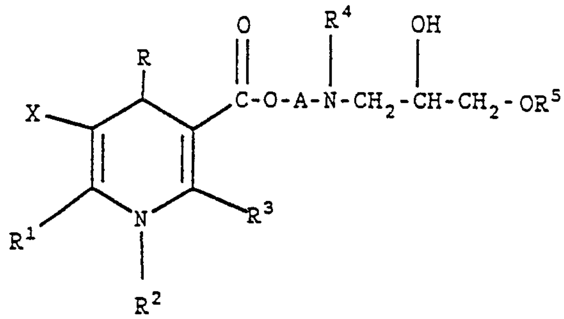

(I)

in welcher

R für Phenyl, Pyridyl oder den Benzoxadiazolylrest steht, wobei der Phenylrest 1 oder 2 gleiche oder verschiedene Substituenten aus der Gruppe Nitro, Trifluormethyl, Fluor, Chlor oder Cyano enthält,

$R^1$ und $R^3$ gleich oder verschieden sind une jeweils für Alkyl mit 1 bis 4 Kohlenstoffatomen stehen oder einer der Substituenten $R^1$ oder $R^3$ für Hydroxymethyl, Amino oder Cyano steht,

$R^2$ für Wasserstoff oder Alkyl mit 1 bis 2 Kohlenstoffatomen steht,

A entweder für eine durch Methyl, Ethyl oder Propyl substituierte Alkylengruppe mit bis zu 12 Kohlenstoffatomen steht, die gegebenenfalls durch eine Phenylgruppe substituiert oder unterbrochen ist und/oder die gegebenenfalls durch ein Sauerstoff- oder Schwefelatom oder eine Gruppierung der Formel $—NR^6$ unterbrochen ist,

oder

A für eine geradkettige oder verzweigte Alkylengruppe mit bis zu 12 Kohlenstoffatomen steht, die gegebenenfalls durch 1 Sauerstoff- oder Schwefelatom oder eine Gruppierung der Formel $—NR^6$ unterbrochen ist, wenn

a) $R^4$ für Phenyl, Benzyl, $CO—R^6$, $CO_2—R^6$, $CO—NR^6R^7$, $SO_2R^6$ oder $—CH_2—CHOH—CH_2—OR^8$ steht, oder

b) R für Pyridyl oder Benzoxadiazolyl steht, oder

c) $R^5$ für Indolyl steht, oder

d) die Alkylenkette durch ein Schwefelatom oder die Gruppe $—NR^6$ unterbrochen ist oder

e) X für $COR^9$ steht,

wobei

$R^6$ und $R^7$ gleich oder verschieden sind und jeweils für Wasserstoff, Alkyl mit bis zu 4 C-Atomen, Benzyl oder Phenyl stehen und wobei

$R^8$ die für $R^5$ angegebene Bedeutung hat und mit diesen gleich oder verschieden sein kann und wobei

$R^9$ für Alkyl mit 1 bis 7 C-Atomen steht, welches gegebenenfalls durch Halogen, Cyano, Alkoxy oder Dialkylamino mit jeweils 1-4 C-Atomen substituiert ist, oder Phenyl, Naphthyl, Phenylalkyl oder Anilino oder eine Amino-, Monoalkylamino- oder Dialkylaminogruppe mit je 1 bis 4 C-Atomen bedeutet, wobei gegebenenfalls die Alkylgruppen mit dem Stickstoffatom einen 5- bis 7-gliedrigen Ring bilden, der als weiteres Heteroatom ein Sauerstoffatom enthalten kann und wobei

$R^{6'}$ die Bedeutung von $R^6$ hat jedoch nicht Wasserstoff bedeutet,

$R^4$ für Wasserstoff, für geradkettiges oder verzweigtes Alkyl mit 1 bis 3 Kohlenstoffatomen, für Phenyl, Benzyl oder für einen Rest der Formel $CO—R^6$, $CO_2—R^{6'}$, $CO—NR^6R^7$, $SO_2R^6$ oder $—CH_2—CHOH—CH_2—OR^8$ steht, wobei

$R^6$, $R^{6'}$, $R^7$, $R^8$ und $R^9$ die oben angegebene Bedeutung haben,

$R^5$ für Phenyl, Naphthyl oder Indolyl steht, wobei der Phenylrest gegebenenfalls durch 1 oder 2 gleiche oder verschiedene Substituenten aus der Gruppe Fluor, Chlor, Nitro, Cyano, Alkyl mit 1 bis 2 C-Atomen, $NR^6$, $R^7$, $NR^6COR^7$, die Gruppe $CH_2—CO—NH_2$ oder Acetyl substituiert ist und

X für die Gruppe $COOR^{10}$ steht, wobei $R^{10}$ für einen geradkettigen, verzweigten oder cyclischen

Alkylrest mit bis zu 12 C-Atomen steht, der gegebenenfalls durch ein Sauerstoffatom in der Kette unterbrochen ist und/oder der gegebenenfalls substituiert ist durch Fluor, Chlor, Hydroxy, Pyridyl, Phenyl oder Amino oder für die Gruppe $COR^9$ steht, worin $R^9$ die oben angegebene Bedeutung hat,
sowie deren pharmazeutisch unbedenklichen Säureadditionssalze,
dadurch gekennzeichnet, daß man Dihydropyridinamine der allgemeinen Formel II

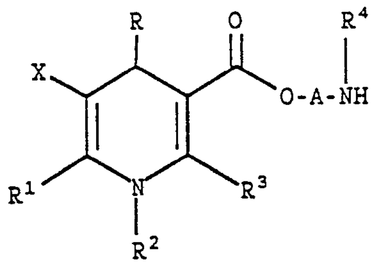

(II)

in welcher
R, $R^1$, $R^2$, $R^3$, X und A die oben angegebene Bedeutung haben und
$R^{4'}$ für Wasserstoff, Alkyl mit 1 bis 6 C-Atomen, Phenyl oder Benzyl steht,
mit Epoxiden der allgemeinen Formel III in welcher

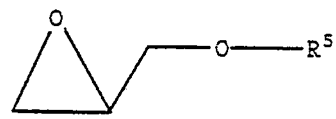

(III)

$R^5$ die oben angegebene Bedeutung hat,
gegebenenfalls in Gegenwart von inerten organischen Lösungsmitteln bei Temperaturen zwischen 10 und 150 °C umsetzt und anschließend gegebenenfalls in ihre Säureadditionssalze überführt.

4. Arzneimittel enthaltend mindestens eine Verbindung gemäß Anspruch 1 oder 2.

5. Verfahren zur Herstellung von Arzneimitteln, dadurch gekennzeichnet, daß man einen Wirkstoff gemäß den Ansprüchen 1 oder 2 gegebenenfalls unter Verwendung von üblichen Hilfs- oder Trägerstoffen in eine geeignete Applikationsform überführt.

6. Verbindungen gemäß Anspruch 1 und 2 zur Bekämpfung von Kreislauferkrankungen.

7. Verwendung von Verbindungen gemäß Anspruch 1 und 2 zur Herstellung von Arzneimitteln mit Kreislaufwirkung.

**Claims**

1. Compounds of the general formula (I)

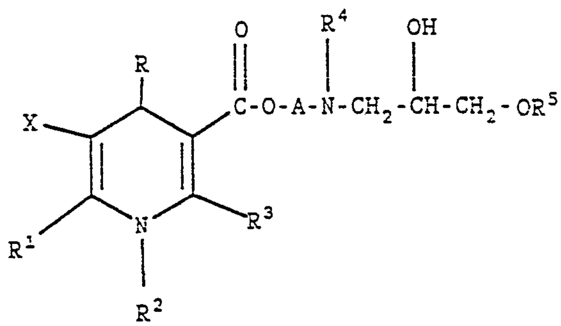

(I)

in which
R represents phenyl, pyridyl or the benzoxadiazolyl radical, the phenyl radical containing 1 or 2 identical or different substituents from the group comprising nitro, trifluoromethyl, fluorine, chlorine and cyano,
$R^1$ and $R^3$ are identical of different and each represent alkyl with 1 to 4 carbon atoms, or one of the substituents $R^1$ or $R^3$ represents hydroxymethyl, amino or cyano,
$R^2$ represents hydrogen or alkyl with 1 or 2 carbon atoms,
A either represents an alkylene group which has up to 12 carbon atoms and is substituted by methyl, ethyl or propyl, and which is optionally substituted or interrupted by a phenyl group and/or is optionally interrupted by an oxygen or sulphur atom or a grouping of the formula $—NR^6$, or

A represents a straight-chain or branched alkylene group which has up to 12 carbon atoms and is optionally interrupted by an oxygen or sulphur atom or a grouping of the formula —$NR^6$
when

a) $R^4$ represents phenyl, benzyl, CO—$R^6$, $CO_2$—$R^{6'}$, CO—$NR^6R^7$, $SO_2R^6$ or —$CH_2$—CHOH—$CH_2$—$OR^8$, or
b) R represents pyridyl or benzoxadiazolyl, or
c) $R^5$ represents indolyl, or
d) the alkylene chain is interrupted by a sulphur atom or the group —$NR^6$ or
e) X represents $COR^9$,

wherein
$R^6$ und $R^7$ are identical or different and each represent hydrogen, alkyl with up to 4 C atoms, benzyl or phenyl and
wherein
$R^8$ has the meaning given for $R^5$ and can be identical or different from this radical
wherein
$R^9$ represents alkyl which has 1 to 7 C atoms and is optionally substituted by halogen, cyano, alkoxy or dialkylamino with in each case 1-4 C atoms, or denotes phenyl, naphthyl, phenylalkyl or anilino or an amino, monoalkylamino or dialkylamino group with in each case 1 to 4 C atoms, the alkyl groups optionally forming, with the nitrogen atom a 5-membered to 7-membered ring which can contain an oxygen atom as a further hetero-atom and
wherein
$R^{6'}$ has the meaning of $R^6$, but does not denote hydrogen,
$R^4$ represents hydrogen, or represents straight-chain or branched alkyl with 1 to 3 carbon atoms, or represents phenyl or benzyl, or represents a radical of the formula CO—$R^6$, $CO_2$—$R^{6'}$, CO—$NR^6R^7$, $SO_2$—$R^6$ or —$CH_2$—CHOH—$CH_2$—$OR^8$,
wherein
$R^6$, $R^{6'}$, $R^7$, $R^8$ und $R^9$ have the abovementioned meaning,
$R^5$ represents phenyl, naphthyl or indolyl, the phenyl radical optionally being substituted by 1 or 2 identical or different substituents from the group comprising fluorine, chlorine, nitro, cyano, alkyl with 1 or 2 C atoms, $NR^6$, $R^7$, $NR^6COR^7$, the group $CH_2$—CO—$NH_2$ or acetyl and
X represents the group $COOR^{10}$, wherein $R^{10}$ represents a straight-chain, branched or cyclic alkyl radical which has up to 12 C atoms and is optionally interrupted in the chain by an oxygen atom and/or is optionally substituted by fluorine, chlorine, hydroxyl, pyridyl, phenyl or amino, or
represents the group $COR^9$, wherein $R^9$ has the abovementioned meaning,
and pharmaceutically acceptable acid addition salts thereof.

2. Compound of the formula

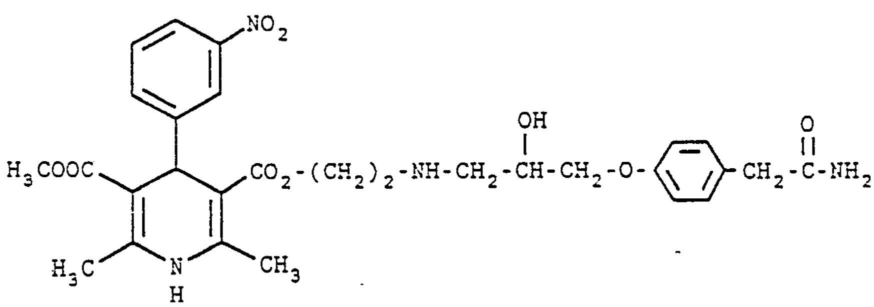

3. Process for the preparation of compounds of the general formula

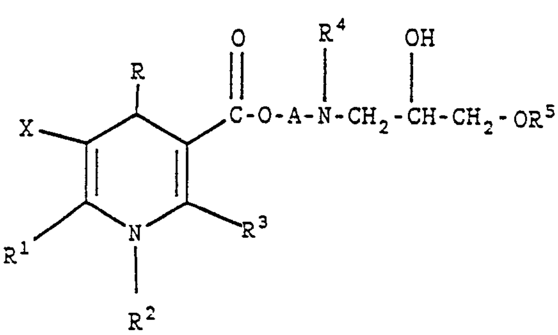

(I)

in which
R represents phenyl, pyridyl or the benzoxadiazolyl radical, the phenyl radical containing 1 or 2

identical or different substituents from the group comprising nitro, trifluoromethyl, fluorine, chlorine and cyano,

$R^1$ and $R^3$ are identical or different and each represent alkyl with 1 to 4 carbon atoms, or one of the substituents $R^1$ or $R^3$ represents hydroxymethyl, amino or cyano,

$R^2$ represents hydrogen or alkyl with 1 or 2 carbon atoms,

A either represents an alkylene group which has up to 12 carbon atoms and is substituted by methyl, ethyl or propyl, and which is optionally substituted or interrupted by a phenyl group and/or is optionally interrupted by an oxygen or sulphur atom or a grouping of the formula $—NR^6$, or

A represents a straight-chain or branched alkylene group which has up to 12 carbon atoms and is optionally interrupted by an oxygen or sulphur atom or a grouping of the formula $—NR^6$ when

a) $R^4$ represents phenyl, benzyl, $CO—R^6$, $CO_2—R^{6'}$, $CO—NR^6R^7$, $SO_2R^6$ or $—CH_2—CHOH—CH_2—OR^8$, or

b) R represents pyridyl or benzoxadiazolyl, or

c) $R^5$ represents indolyl, or

d) the alkylene chain is interrupted by a sulphur atom or the group $—NR^6$ or

e) X represents $COR^9$,

wherein

$R^6$ and $R^7$ are identical or different and each represent hydrogen, alkyl with up to 4 C atoms, benzyl or phenyl and

wherein

$R^8$ has the meaning given for $R^5$ and can be identical or different from this radical

wherein

$R^9$ represents alkyl which has 1 to 7 C atoms and is optionally substituted by halogen, cyano, alkoxy or dialkylamino with in each case 1-4 C atoms, or denotes phenyl, naphthyl, phenylalkyl or anilino or an amino, monoalkylamino or dialkylamino group with in each case 1 to 4 C atoms, the alkyl groups optionally forming, with the nitrogen atom a 5-membered to 7-membered ring which can contain an oxygen atom as a further hetero-atom and

wherein

$R^{6'}$ has the meaning of $R^6$, but does not denote hydrogen,

$R^4$ represents hydrogen, or represents straight-chain or branched alkyl with 1 to 3 carbon atoms, or represents phenyl or benzyl, or represents a radical of the formula $CO—R^6$, $CO_2—R^{6'}$, $CO—NR^6R^7$, $SO_2R^6$ or $—CH_2—CHOH—CH_2—OR^8$,

wherein

$R^6$, $R^{6'}$, $R^7$, $R^8$ and $R^9$ have the abovementioned meaning,

$R^5$ represents phenyl, naphthyl or indolyl, the phenyl radical optionally being substituted by 1 or 2 identical or different substituents from the group comprising fluorine, chlorine, nitro, cyano, alkyl with 1 or 2 C atoms, $NR^6$, $R^7$, $NR^6COR^7$, the groupe $CH_2—CO—NH_2$ or acetyl and

X represents the group $COOR^{10}$, wherein $R^{10}$ represents a straight-chain, branched or cyclic alkyl radical which has up to 12 C atoms and is optionally interrupted in the chain by an oxygen atom and/or is optionally substituted by fluorine, chlorine, hydroxyl, pyridyl, phenyl or amino, or

represents the group $COR^9$, wherein $R^9$ has the abovementioned meaning,

and pharmaceutically acceptable acid addition salts thereof,

characterized in that dihydropyridineamines of the general formula II

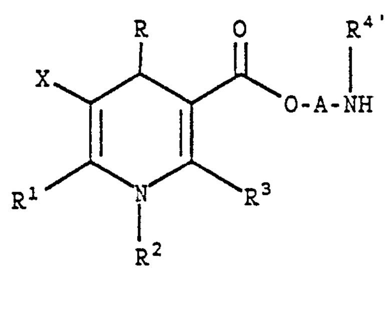

(II)

in which

R, $R^1$, $R^2$, $R^3$, X and A have the abovementioned meaning and

$R^{4'}$ represents hydrogen, alkyl with 1 to 6 C atoms, phenyl, or benzyl,

are reacted with epoxides of the general formula III

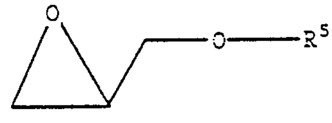

(III)

in which

$R^5$ has the abovementioned meaning,

if appropriate in the presence of inert organic solvents at temperatures between 10 and 150 °C and, if appropriate, the products are then converted into their acid addition salts.

4. Medicaments containing at least one compound according to Claim 1 or 2.

5. Process for the preparation of medicaments, characterized in that an active compound according to Claims 1 or 2 is converted into a suitable administration form, if appropriate using customary auxiliaries and/or excipients.

6. Compounds according to Claim 1 and 2 for combating circulatory diseases.

7. Use of compounds according to Claim 1 and 2 for the preparation of medicaments with a circulatory action.

**Revendications**

1. Composés de formule générale I

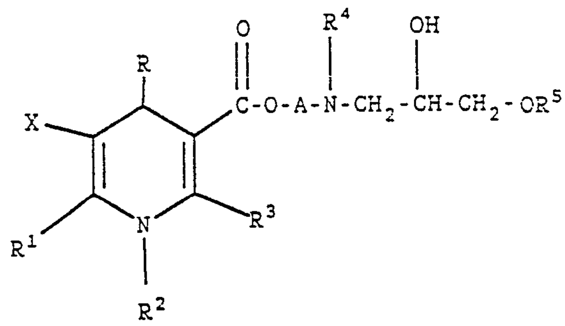

(I)

dans laquelle

R représente un groupe phényle, pyridyle ou benzoxadiazolyle, le groupe phényle portant un ou deux substituants identiques ou différents choisis parmi les groupes nitro, trifluorométhyle, le fluor, le chlore ou les groupes cyano,

$R^1$ et $R^3$, ayant des significations identiques ou différentes, représentent chacun un groupe alkyle en $C_1$-$C_4$, ou bien l'un des symboles $R^1$ ou $R^3$ représente un groupe hydroxyméthyle, amino ou cyano,

$R^2$ représente l'hydrogène ou un groupe alkyle en $C_1$-$C_2$,

A représente soit un groupe alkylène contenant jusqu'à 12 atomes de carbone, substitué par des groupes méthyle, éthyle ou propyle, éventuellement substitué ou interrompu par un groupe phényle et/ou éventuellement interrompu par un atome d'oxygène ou de soufre ou un groupement de formule —$NR^6$,

soit un groupe alkylène à chaîne droite ou ramifiée contenant jusqu'à 12 atomes de carbone, éventuellement interrompu par un atome d'oxygène ou de soufre ou par un groupement de formule —$NR^6$ lorsque

a) $R^4$ représente un groupe phényle, benzyle, CO—$R^6$, $CO_2$—$R^{6'}$, CO—$NR^6R^7$, $SO_2R^6$ ou —$CH_2$—CHOH—$CH_2$—$OR^8$, ou bien

b) R représente un groupe pyridyle ou benzoxadiazolyle, ou bien

c) $R^5$ représente un groupe indolyle, ou bien

d) la chaîne alkylène est interrompue par un atome de soufre ou par le groupe —$NR^6$, ou bien

e) X représente un groupe $COR^9$,

$R^6$ et $R^7$, ayant des significations identiques ou différentes, représentant chacun l'hydrogène, un groupe alkyle contenant jusqu'à 4 atomes de carbone, benzyle ou phényle, et

$R^8$ a les significations indiquées pour $R^5$ mais peut être identique à celui-ci ou différent de celui-ci, et

$R^9$ représente un groupe alkyle en $C_1$-$C_7$ éventuellement substitué par des halogènes, des groupes cyano, alcoxy ou dialkylamino contenant chacun 1 à 4 atomes de carbone, ou un groupe phényle, naphtyle, phénylalkyle ou anilino ou un groupe amino, monoalkylamino ou dialkylamino contenant chacun 1 à 4 atomes de carbone, les groupes alkyle pouvant éventuellement former avec l'atome d'azote un cycle de 5 à 7 chaînons qui peut contenir un atome d'oxygène en tant qu'autre hétéroatome, et

$R^{6'}$ a les significations indiquées pour $R^6$ à l'exception de l'hydrogène,

$R^4$ représente l'hydrogène, un groupe alkyle à chaîne droite ou ramifiée en $C_1$-$C_3$, un groupe phényle, benzyle ou un groupe de formule CO—$R^6$, $CO_2$—$R^{6'}$, CO—$NR^6R^7$, $SO_2R^6$ ou —$CH_2$—CHOH—$CH_2$—$OR^8$, dans lesquels

$R^6$, $R^{6'}$, $R^7$, $R^8$ et $R^9$ ont les significations indiquées ci-dessus,

$R^5$ représente un groupe phényle, naphtyle ou indolyle, le groupe phényle pouvant éventuellement porter 1 ou 2 substituants identiques ou différents choisis parmi le fluor, le chlore, les groupes nitro, cyano, alkyle en $C_1$-$C_2$, $NR^6$, $R^7$, $NR^6COR^7$, le groupe $CH_2$—CO—$NH_2$ ou un groupe acétyle, et

X représente le groupe $COOR^{10}$ dans lequel $R^{10}$ représente un groupe alkyle à chaîne droite, ramifiée ou cyclique contenant jusqu'à 12 atomes de carbone, éventuellement interrompu dans la chaîne

par un atome d'oxygène et/ou éventuellement substitué par le fluor, le chlore, les groupes hydroxy, pyridyle, phényle ou amino, ou bien le groupe $COR^9$ dans lequel $R^9$ a les significations indiquées ci-dessus,

et leurs sels formés par addition avec des acides acceptables pour l'usage pharmaceutique.

2. Composé de formule

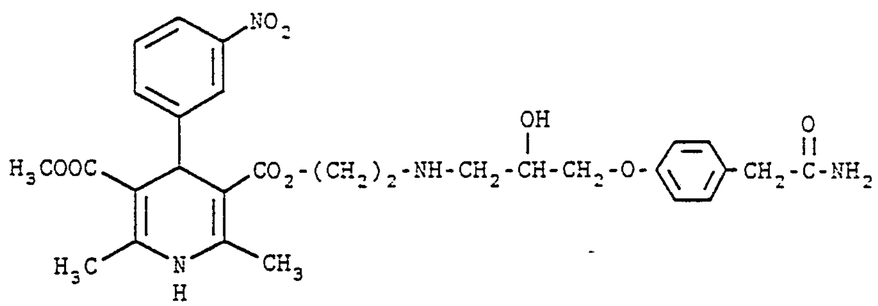

3. Procédé de préparation des composés de formule générale I

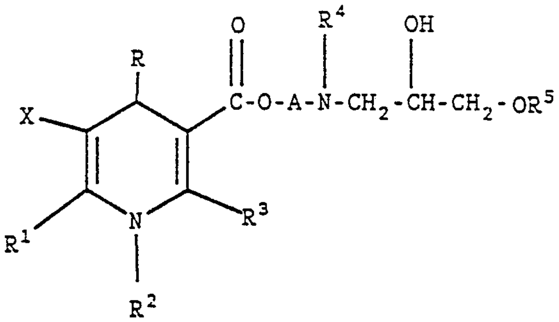

(I)

dans laquelle

R représente un groupe phényle, pyridyle ou benzoxadiazolyle, le groupe phényle portant 1 ou 2 substituants identiques ou différents choisis parmi les groupes nitro, trifluorométhyle, le fluor, le chlore ou les groupes cyano,

$R^1$ et $R^3$, ayant des significations identiques ou différentes, représentent chacun un groupe alkyle en $C_1$-$C_4$, ou bien l'un des substituants $R^1$ ou $R^3$ représente un groupe hydroxyméthyle, amino ou cyano,

$R^2$ représente l'hydrogène ou un groupe alkyle en $C_1$-$C_2$,

A représente soit un groupe alkylène contenant jusqu'à 12 atomes de carbone, substitué par des groupes méthyle, éthyle ou propyle, éventuellement substitué ou interrompu par un groupe phényle et/ou éventuellement interrompu par un atome d'oxygène ou de soufre ou un groupement de formule $—NR^6$,

soit un groupe alkylène à chaîne droite ou ramifiée contenant jusqu'à 12 atomes de carbone, éventuellement interrompu par un atome d'oxygène ou de soufre ou par un groupement de formule $—NR^6$ lorsque

a) $R^4$ représente un groupe phényle, benzyle, $CO—R^6$, $CO_2—R^{6'}$, $CO—NR^6R^7$, $SO_2R^6$ ou $—CH_2—CHOH—CH_2—OR^8$, ou bien

b) R représente un groupe pyridyle ou benzoxadiazolyle, ou bien

c) $R^5$ représente un groupe indolyle, ou bien

d) la chaîne alkylène est interrompue par un atome de soufre ou par le groupe $—NR^6$, ou bien

e) X représente un groupe $COR^9$,

$R^6$ et $R^7$, ayant des significations indentiques ou différentes, représentant chacun l'hydrogène, un groupe alkyle contenant jusqu'à 4 atomes de carbone, benzyle ou phényle, et

$R^8$ a les significations indiquées pour $R^5$ mais est identique à celui-ci ou différent de celui-ci, et

$R^9$ représente un groupe alkyle en $C_1$-$C_7$ éventuellement substitué par des halogènes, des groupes cyano, alcoxy ou dialkylamino contenant chacun 1 à 4 atomes de carbone, ou un groupe phényle, naphtyle, phénylalkyle ou anilino ou un groupe amino, monoalkylamino ou dialkylamino contenant chacun 1 à 4 atomes de carbone, les groupes alkyle pouvant éventuellement former avec l'atome d'azote un cycle de 5 à 7 chaînons qui peut contenir un atome d'oxygène en tant qu'autre hétéroatome, et

$R^{6'}$ a les significations de $R^6$ à l'exception de l'hydrogène,

$R^4$ représente l'hydrogène, un groupe alkyle à chaîne droite ou ramifiée en $C_1$-$C_3$, un groupe phényle, benzyle ou un groupe de formule $CO—R^6$, $CO_2—R^{6'}$, $CO—NR^6R^7$, $SO_2R^6$ ou $—CH_2—CHOH—CH_2—OR^8$ dans lesquels,

$R^6$, $R^{6'}$, $R^7$, $R^8$ et $R^9$ ont les significations indiquées ci-dessus,

$R^5$ représente un groupe phényle, naphtyle ou indolyle, le groupe phényle portant éventuellement 1

ou 2 substituants identiques ou différents choisis parmi le fluor, le chlore, les groupes nitro, cyano, alkyle en $C_1$-$C_2$, $NR^6$, $R^7$, $NR^6COR^7$, le groupe $CH_2$—CO—$NH_2$ ou un groupe acétyle, et

X représente le groupe $COOR^{10}$ dans lequel $R^{10}$ représente un groupe alkyle à chaîne droite, ramifiée ou cyclique contenant jusqu'à 12 atomes de carbone, qui est éventuellement interrompu dans la chaîne par un atome d'oxygène et/ou qui est éventuellement substitué par le fluor, le chlore, des groupes hydroxy, pyridyle, phényle ou amino, ou bien le groupe $COR^9$ dans lequel $R^9$ a les significations indiquées ci-dessus

et de leurs sels formés par addition avec des acides acceptables pour l'usage pharmaceutique, caractérisé en ce que l'on fait réagir des dihydropyridinamines de formule générale II

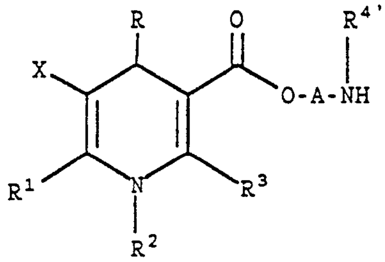

(II)

dans laquelle

R, $R^1$, $R^2$, $R^3$, X et A ont les significations indiquées ci-dessus, et

$R^{4'}$ représente l'hydrogène, un groupe alkyle en $C_1$-$C_6$, phényle ou benzyle, avec des époxydes de formule générale III

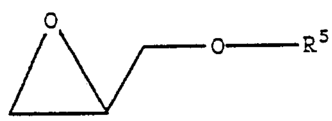

(III)

dans laquelle $R^5$ a les significations indiquées ci-dessus, éventuellement en présence de solvants organiques inertes, à des températures de 10 à 150 °C, en faisant suivre le cas échéant de la conversion en sels par addition avec des acides.

4. Médicament contenant au moins un composé selon la revendication 1 ou 2.

5. Procédé de préparation de médicaments, caractérisé en ce que l'on met une substance active selon les revendications 1 ou 2, éventuellement avec utilisation conjointe de produits auxiliaires ou véhicules usuels, sous une forme d'administration appropriée.

6. Composés selon les revendications 1 et 2, pour le traitement des maladies de la circulation.

7. Utilisation des composés selon les revendications 1 et 2, pour la préparation de médicaments agissant sur la circulation.